# FASCICULE DE BREVET EUROPEEN

(11) **EP 2 925 882 B1**
(45) Date de publication et mention de la délivrance du brevet: **26.04.2017**
(21) Numéro de dépôt: 13799051.1
(22) Date de dépôt: 02.12.2013
(51) Int. Cl.: C12Q 1/68

(54) **MÉTHODE DE CRIBLAGE À HAUT-DÉBIT POUR L'IDENTIFICATION DE BIOMARQUEURS, CIBLES THÉRAPEUTIQUES OU D'AGENTS THÉRAPEUTIQUES**
SCREENING-VERFAHREN MIT HOHEM DURCHSATZ ZUR IDENTIFIZIERUNG VON BIOMARKERN, THERAPEUTISCHEN ZIELEN ODER THERAPEUTISCHEN WIRKSTOFFEN
HIGH-THROUGHPUT SCREENING METHOD FOR THE IDENTIFICATION OF BIOMARKERS, THERAPEUTIC TARGETS OR THERAPEUTIC AGENTS

(30) Priorité: 30.11.2012 FR 1261520
(43) Date de publication de la demande: 07.10.2015
(73) Titulaire: Commissariat à l'Energie Atomique et aux Energies Alternatives, 75015 Paris (FR)
(72) Inventeur: NAVARRO Y GARCIA, Fabrice, 38600 Fontaine (FR); BRUNIAUX, Jonathan, 38000 Grenoble (FR); GIDROL, Xavier, 06570 saint Paul de Vence (FR); SULPICE, Eric, 38330 Biviers (FR)
(74) Mandataire: Lavoix
(86) Numéro de dépôt international: PCT/EP2013/075279
(87) Numéro de publication internationale: WO 2014/083205

(56) Documents cités:
- WO-A1-2012/019765
- WO-A2-2012/006380
- BERNS K ET AL: "A large-scale RNAi screen in human cells identifies new components of the p53 pathway", NATURE: INTERNATIONAL WEEKLY JOURNAL OF SCIENCE (AND SUPPLEMENTARY INFORMATION), NATURE PUBLISHING GROUP, UNITED KINGDOM, vol. 428, 25 mars 2004 (2004-03-25), pages 431-437, XP003002475, ISSN: 0028-0836, DOI: 10.1038/NATURE02371
- XIAN-ZHU YANG ET AL: "Systemic delivery of siRNA with cationic lipid assisted PEG-PLA nanoparticles for cancer therapy", JOURNAL OF CONTROLLED RELEASE, ELSEVIER, AMSTERDAM, NL, vol. 156, no. 2, 26 juillet 2011 (2011-07-26), pages 203-211, XP028112013, ISSN: 0168-3659, DOI: 10.1016/J.JCONREL.2011.07.035 [extrait le 2011-08-01]
- SANTEL A ET AL: "A novel siRNA-lipoplex technology for RNA interference in the mouse vascular endothelium", GENE THERAPY, NATURE PUBLISHING GROUP, GB, vol. 13, no. 16, 20 avril 2006 (2006-04-20), pages 1222-1234, XP002459352, ISSN: 0969-7128, DOI: 10.1038/SJ.GT.3302777

## Description

La présente invention concerne une méthode de criblage d'une molécule d'intérêt à l'aide de nanoparticules comprenant une molécule candidate, un traceur, et une étiquette ADN unique propre à ladite molécule. La présente invention concerne également une méthode de criblage de biomarqueurs d'une maladie et/ou d'un trait phénotypique, plus particulièrement d'un cancer ou d'une infection, à l'aide desdites nanoparticules. L'invention concerne enfin les nanoparticules en tant que telles ainsi qu'une bibliothèque desdites nanoparticules.

La génétique inverse cherche à déterminer la fonction d'un gène donné par l'analyse des conséquences phénotypiques de la mutation de ce gène. Cette approche « s'oppose » (inverse) à la démarche classique qui, pour un mutant phénotypique donné, cherche à identifier le gène responsable. Les cribles de génétique inverse ont ainsi par exemple permis l'identification des régulateurs clés du cycle cellulaire chez la levure (Hartwell et al., 1974, Science, 183:46-51), ou des principes de base des mécanismes du développement des organismes multicellulaires chez la drosophile (Nussleinvolhard et Wieschaus, 1980, Nature, 287:795-801).

Les cribles de génétique inverse peuvent reposer sur des gains de fonction (surexpression d'un gène à l'aide d'un vecteur d'expression, d'un analogue de microRNA, etc...) ou sur des pertes de fonction provoquées par des inhibiteurs chimiques de protéines ou des ARN interférents (ARNi). Les cribles chimiques (approche parfois dénommée chémogénomique) ont permis, *via* l'inhibition des protéines, la découverte de nombreuses molécules aujourd'hui testées en essais cliniques, voire déjà présentes sur le marché en tant que médicament.

L'ARN interférence (ARNi) est un processus naturel, conservé au cours de l'évolution, selon lequel l'introduction d'ARN double brins dans une cellule provoque la dégradation spécifique des séquences homologues de mRNA (Fire et al., 1998, Nature 391:806-811). De même, les microRNAs (miRNA) sont des petits ARN non-codant endogènes, présents dans le génome, qui régulent spécifiquement l'expression de quelques gènes cibles. Il existe de nombreux autres petits ARN non codants (piRNA, natsiRNA, etc...) qui participent à la régulation de l'expression des gènes.

*In fine,* il convient de garder à l'esprit que l'inhibition spécifique d'une protéine par un inhibiteur chimique, la mutation « perte de fonction » d'un gène ou la suppression de l'expression d'une protéine par l'ARNi endogène (miRNA) ou exogène (siRNA) sont fonctionnellement équivalentes.

L'ARNi fournit donc une méthode de génétique inverse extrêmement puissante, spécialement pour les organismes où la génétique classique est difficile comme chez l'homme. A partir des séquences du génome humain, des collections d'ARNi ciblant la totalité des gènes ont été développées, permettant la réalisation de cribles de génétique inverse sur les lignées cellulaires humaines, voire même sur des cellules primaires humaines (Boutros et Ahringer, 2008, Nature Reviews Genetics 9:554-566). Plusieurs études récentes ont montré la puissance des cribles d'ARNi à large échelle pour caractériser la pertinence clinique de gènes particuliers pour la réponse individuelle des patients aux traitements. De plus, avec le développement récent des inhibiteurs de miRNA tels que les oligonucléotides modifiés « locked nucleic acid (LNA) » (Orom et al., 2006, Gene, 372 : 137-141), les oligonucléotides anti-miRNA (AMOs) (Weiler et al., 2006, Gene Therapy, 13 : 496-502) et les "antagomirs" (Krutzfeldt et al., 2005, Nature, 438 : 685-689), des cribles génétiques basés sur la perte de fonction des miRNA sont aussi possibles. Par ailleurs, le développement des analogues de miRNA, de piRNA, etc... rend également possible des cribles fonctionnels à haut débit en gain de fonction avec ces molécules. Berns et al., 2004, Nature, 428 divulgue une méthode de criblage pour identifier un petit ARN interfèrent (siARN) d'intérêt.

A ce jour la très grande majorité des cribles cellulaires à haut débit utilisent des plaques ou puces de microtitration à 96 ou à 384 puits qui sont lues (capturées) par un microscope automatisé ou un lecteur spectrophotométrique de plaques (UV, visible, IR).

Cependant cette approche en plaque ou en puce souffre de plusieurs limitations. Premièrement, les plaques comme les puces nécessitent un ordonnancement préalable des molécules candidates (collections d'agents chimiques ou d'acides nucléiques) qui seront utilisées au cours du crible et un suivi minutieux de cet ordonnancement pour retrouver les molécules à l'origine du phénotype étudié. Deuxièmement, il est difficile, de réaliser ces cribles sur des cellules en suspension (comme les cellules sanguines), des cellules en culture 3D, des populations cellulaires mixtes, des cellules en co-culture ou des cellules nécessitant une couche nourricière (feeder layer en anglais). Troisièmement, le suivi des cellules ayant incorporé la molécule candidate est difficile car il nécessite un marquage préalable de chacune de ces molécules chimiques ou génétiques, ce marquage pouvant altérer leur efficacité. Quatrièmement, ces formats nécessitent des méthodes d'analyse d'image complexes tant au niveau algorithmique, que pour le stockage et la sauvegarde des données. Cinquièmement, la récupération des cellules après analyse est très difficile, voire impossible. Sixièmement, et en corollaire de la limitation précédente, la surface de culture disponible dans les plaques ou les puces limite les phénotypes étudiés (pas plus de 72h). L'analyse de phénotypes nécessitant plus de temps, comme par exemple la différenciation dans un lignage particulier, est impossible sans récupérer les cellules et les transférer dans un autre format de culture.

Pour répondre à quelques-unes de ces limitations, il a été proposé d'utiliser un système de code-barre nucléique porté par des vecteurs viraux pour identifier en aval, avec des puces à ADN dédiées, les acides nucléiques responsables du phénotype étudié (Berns et al., 2004, Nature, 428:431-7). Plusieurs évolutions de ce concept ont depuis été proposées (Verzijlbergen et al., 2011, PLoS Genet, 10:e1002284). Cependant, cette approche présente elle aussi plusieurs limitations. Premièrement, elle n'est pas envisageable pour les molécules chimiques. Deuxièmement, développée pour les ARN interférents transcrits et maturés par la machinerie cellulaire, elle ne fonctionne pas avec d'autres types d'acides nucléiques, non maturés par la cellule comme les LNA ou des analogues de miRNA, de piRNA, etc. Troisièmement, elle repose sur l'utilisation de virus et donc nécessite des manipulations en laboratoire au moins de type L2 et idéalement de type L3. Quatrièmement, l'utilisation d'une séquence modifiée par un code-barre (i.e une étiquette ADN) risque d'intercéder dans le processus d'ARN interférence et il n'est pas certain que la maturation de l'ARNi et l'efficacité d'extinction soit systématiquement conservée avec ces constructions virales. Cinquièmement, l'infection virale peut elle-même induire des modifications phénotypiques indépendantes de la molécule candidate.

Le développement de méthodes de criblage permettant de pallier ces limitations est donc nécessaire.

Les inventeurs ont ainsi développé une approche sans ordonnancement préalable des molécules candidates, associée à une lecture phénotypique au niveau de la cellule individuelle et à un tri à très haut débit des cellules d'intérêt, basée sur l'utilisation de nanoparticules permettant de « délivrer » simultanément, au sein d'une cellule, une molécule candidate, un traceur permettant de suivre spécifiquement les cellules ayant incorporé la molécule candidate, et une étiquette ADN unique propre à la molécule candidate permettant d'identifier la molécule candidate *a posteriori,* après lecture du phénotype, par déconvolution de l'étiquette ADN unique. La capacité de « délivrance » simultanée de la molécule candidate, du traceur et de l'étiquette ADN par la nanoparticule est notamment due à la taille (inférieure à 1 micron, et de préférence inférieure à 200nm) et à la charge (de préférence de charge positive) de la nanoparticule ce qui confère à celle-ci une efficacité de pénétration importante dans les cellules.

A cet effet, selon un premier objet, la présente invention fournit une méthode de criblage d'une molécule d'intérêt comprenant les étapes suivantes :
a) mettre en contact des cellules avec une bibliothèque de nanoparticules, chaque nanoparticule comprenant une molécule candidate, un traceur et une étiquette ADN unique propre à ladite molécule candidate, dans des conditions permettant auxdites cellules d'intégrer au moins une desdites nanoparticules,
b) sélectionner les cellules ayant intégré le traceur et présentant un phénotype d'intérêt,
c) identifier en tant que molécule d'intérêt la molécule candidate ayant été intégrée dans la cellule sélectionnée à l'étape b) en identifiant la séquence de ladite étiquette ADN unique.

La présente invention concerne également une méthode de criblage d'un biomarqueur d'une maladie ou d'un trait phénotypique comprenant les étapes suivantes :
a) mettre en contact des cellules avec une bibliothèque de nanoparticules, chaque nanoparticule comprenant une molécule candidate, un traceur et une étiquette ADN unique propre à ladite molécule candidate, dans des conditions permettant auxdites cellules d'intégrer au moins une desdites nanoparticules,
b) sélectionner les cellules ayant intégré le traceur et présentant un phénotype d'intérêt,
c) identifier la molécule candidate ayant été intégrée dans la cellule sélectionnée à l'étape b) en identifiant la séquence de ladite étiquette ADN unique,
d) identifier en tant que biomarqueur d'une maladie et/ou d'un phénotype la cible de ladite molécule identifiée à l'étape c).

Optionnellement, lesdites méthodes de criblage d'une molécule d'intérêt ou d'un biomarqueur d'une maladie ou d'un trait phénotypique comprennent, avant l'étape a), une étape a0) de préparation d'une bibliothèque de nanoparticules, chaque nanoparticule comprenant une molécule candidate, un traceur et une étiquette ADN unique propre à ladite molécule candidate.

L'invention concerne également l'utilisation d'une nanoparticule comprenant une molécule candidate, un traceur et une étiquette ADN unique propre à ladite molécule candidate pour le criblage de molécules d'intérêt et/ou de biomarqueur d'une maladie et/ou d'un phénotype.

Selon un autre objet, l'invention concerne une nanoparticule comprenant une molécule candidate, un traceur et une étiquette ADN unique propre à ladite molécule candidate.

L'invention concerne également une bibliothèque de nanoparticules, chacune desdites nanoparticules comprenant une molécule candidate, un traceur et une étiquette ADN unique propre à ladite molécule candidate.

### Nanoparticule comprenant une molécule candidate, un traceur et une étiquette ADN unique propre à ladite molécule candidate

L'invention concerne une nanoparticule comprenant une molécule candidate, un traceur et une étiquette ADN unique propre à ladite molécule candidate.

La nanoparticule peut être une nanoparticule neutre, anionique ou cationique. De préférence, ladite nanoparticule est une nanoparticule cationique. La nanoparticule est notamment synthétique. L'utilisation d'une nanoparticule synthétique permet d'effectuer des manipulations peu contraignantes, par rapport à des manipulations mettant en oeuvre des microorganismes ou des virus. Dans certains modes de réalisation, ladite nanoparticule est une nanoparticule inorganique, comme par exemple une nanoparticule d'or, une nanoparticule magnétique (nanoparticule d'oxyde de fer), ou un nanocristal de matériau semi-conducteur (« quantum dot » en anglais). Dans certains modes de réalisation, ladite nanoparticule est une nanoparticule organique telle que par exemple un vecteur cationique. Des exemples de nanoparticules utilisables dans l'invention sont notamment décrits dans Morille et al., 2008, Biomaterials, 29 :3477-3496 ; Bruno, 2011, Advanced Drug Delivery Reviews, 63 :1210-1226 ; Zhang et al., 2012, Bioorganic Chemistry, 40 :10-18. Dans certains modes de réalisation, ladite nanoparticule est une nanoparticule hybride, *i.e.* une nanoparticule comportant un coeur inorganique recouvert d'une couche polymérique.

Dans un mode de réalisation, ledit vecteur cationique est formulé à partir d'au moins un polymère. Des exemples de polymères sont le Polyéthylèneimine (PEI), la poly(L-lysine) (PLL), le poly(α-[4-amino-butyl]-L-glycolique acide), le chitosan, tel que le chitosan galactosylé, le chitosan-graft-poly(vinylpyrrolidone) (PVP) galactosylé, des oligomères de chitosan trimétylés, le chitosan N-dodécylé, le déoxycholique acide chitosan modifié, la polyamidoamine (PAMAM), le poly(acide lactique) (PLA pour Poly(lactic acid) en anglais), le poly(acide lactique-*co*-glycolique) (PLGA pour poly(lactic-co-glycolic acid) en anglais), le polyalkylcyanoacrylate (PACA), les dérivés de cyanoacrylate, tels que le polybutylcyanoacrylate (PBCA), le polyisobutylcyanoacrylate (PIBCA), le polyisohexylcyanoacrylate (PIHCA), le polyhexylcyanoacrylate (PHCA), ou l'isobutylcyanoacrylate (IBCA). Optionnellement, ledit vecteur peut en outre comprendre au moins un poly-ethylène-glycol (PEG).

Dans un mode de réalisation, ledit vecteur cationique est un vecteur lipidique cationique, c'est-à-dire un dérivé cationique ou polycationique de nature lipidique capable d'interagir par effet électrostatique avec une molécule candidate chargée négativement. De préférence, ledit vecteur lipidique cationique est un liposome, ou une gouttelette d'une formulation sous forme d'une nanoémulsion comprenant une phase aqueuse continue et au moins une phase dispersée.

De préférence, ledit liposome comprend ou consiste en au moins un lipide aliphatique monovalent, tel que le 1,2-dioleyl-3-trimethylamonium-propane (DOTAP), le *N*[1-(2,3-dioléyloxy) propyl]-*N,N,N*-triméthylammonium (DOTMA), ou le *N-*(2-hydroxyethyl)-*N,N*-dimethyl-2,3-bis(tetradecyloxy-1-propananium) (DMRIE), un lipide aliphatique multivalent, tel que le dioctadecylamidoglycylspermine (DOGS), ou un dérivé cationique du cholestérol, tel que le 3β-[N-(*N',N*-diméthyl-aminoéthane)-carbamoyl]cholestérol (DC-Chol) ou le bis-guanidium-tren-cholestérol (BGTC). Optionnellement, ledit liposome peut comprendre en outre un lipide fusogène, qui est susceptible de faciliter la libération cytosolique par déstabilisation de la membrane endosomale, dit lipide « helper », tel que le dioléylphosphatidyléthanolamine (DOPE) ou le cholestérol. Ledit liposome peut comprendre au moins 2, 3, 4, 5 lipides tels que décrits ci-dessus. A titre d'exemples non limitatifs, ledit liposome peut donc être un liposome DOTMA/DOPE ou DOTAP/cholestérol. Optionnellement, ledit vecteur peut en outre comprendre au moins un poly-ethylène-glycol (PEG). Des exemples de liposomes comprenant en outre au moins un PEG sont des liposomes constitués de *N,N*-dioleyl-*N,N-*dimethylammonium chloride, de DOPE et de céramides conjugués un PEG, des liposomes constitués de 3-*N*-[methoxypoly(ethyleneglycol)₂₀₀₀)carbamoyl]-1,2dimyristoyloxy-propylamine (PEG-C-DMA), 1,2-dilinoleylloxy-*N-N-*dimethym-3-aminopropane (DLinDMA), 1,2-distearoyl-*sn*-glycero-3-phosphocholine (DSPC) et de cholestérol à un rapport 2:40:10:48 pour cent molaires, des liposomes constitués de β-L-arginyl-2,3-L-diaminopropionic acid-N-palmityl-N-oleyl-amide trihydrochloride, 1,2-diphytanoyl-sn-glycero-3-phosphoethanolamine (DphyPE et le lipide PEGylé N-(carbonyl methyxypolyethyleneglycol-2000)-1,2-distearoyl-sn-glycero-3-phospho-ethanolamine sodium (DSPE-PEG), des liposomes constitués de phosphatidyl choline hydrogénée de soja, de cholestérol, de DSPE-PEG2000 et de DOTAP.

De préférence, ladite gouttelette d'une formulation sous forme de nanoémulsion, comprenant une phase aqueuse continue et au moins une phase dispersée, est telle que définie dans la demande FR 12 58115. Selon ce mode de réalisation, ladite gouttelette d'une formulation sous forme de nanoémulsion, comprenant une phase aqueuse continue et au moins une phase dispersée comprend :
a) au moins 5% molaire de lipide amphiphile,
b) de 15 à 70% molaire d'au moins un tensioactif cationique comprenant :
   i) au moins un groupe lipophile choisi parmi :
      - un groupe R ou R-(C=O)-, où R représente une chaîne hydrocarbonée linéaire comprenant de 11 à 23 atomes de carbone,
      - un ester ou un amide d'acides gras comprenant de 12 à 24 atomes de carbone et de phosphatidyléthanolamine, et
      - un poly(oxyde de propylène), et
   ii) au moins un groupe hydrophile comprenant au moins un groupe cationique choisi parmi :
      - un groupe alkyle linéaire ou ramifié comprenant de 1 à 12 atomes de carbone et interrompu et/ou substitué par au moins un groupe cationique, et
      - un groupe polymérique hydrophile comprenant au moins un groupe cationique, et
c) de 10% à 55% molaire d'un co-tensioactif comprenant au moins une chaîne poly(oxyde d'éthylène) comprenant au moins 25 unités d'oxyde d'éthylène,
d) un lipide solubilisant comprenant au moins un glycéride d'acides gras,
e) éventuellement un lipide fusogène,
où les pourcentages molaires de lipide amphiphile, de tensioactif cationique et de co-tensioactif sont par rapport à l'ensemble (lipide amphiphile / tensioactif cationique / co-tensioactif / éventuel lipide fusogène).

Comme expliqué ci-après, les : lipide amphiphile / tensioactif cationique / co-tensioactif / éventuel lipide fusogène sont les principaux composants de la couronne des gouttelettes de la formulation.

L'émulsion est donc une émulsion de type huile dans l'eau. Elle peut être simple ou multiple, notamment en comportant dans la phase dispersée une seconde phase aqueuse. De préférence, elle est simple.

De plus, la formulation est avantageusement stable : elle peut être stockée plusieurs heures ou semaines sans qu'aucune dégradation ne soit observée.

Ladite formulation est particulièrement adaptée pour être complexée à des molécules chargées négativement, comme par exemple des séquences nucléotidiques susceptible de moduler des mécanismes endogènes d'ARN interférents.

### • Tensioactif cationique

La formulation utilisée dans l'invention comprend un tensioactif cationique comprenant :
a) au moins un groupe lipophile choisi parmi :
   i) un groupe R représentant une chaîne hydrocarbonée linéaire comprenant de 11 à 23 atomes de carbone,
   ii) un ester ou un amide d'acides gras comprenant de 12 à 24 atomes de carbone et de phosphatidyléthanolamine, tel que le distéaryl phosphatidyléthanolamine (DSPE), et
   iii) un poly(oxyde de propylène), et
b) au moins un groupe hydrophile comprenant au moins un groupe cationique choisi parmi :
   i) un groupe alkyle linéaire ou ramifié comprenant de 1 à 12 atomes de carbone et interrompu et/ou substitué par au moins un groupe cationique, et
   ii) un groupe polymérique hydrophile comprenant au moins un groupe cationique, ledit groupe polymérique étant notamment choisi parmi :
      - un poly(oxyde d'éthylène) comprenant typiquement de 3 à 500 unités oxyde d'éthylène, de préférence de 20 à 200 unités oxyde d'éthylène, et comprenant au moins un groupe cationique.
      - un polysaccharide, tel que du dextrane, de la cellulose ou du chitosan, ayant notamment des masses moléculaires comprises entre 0,5 et 20 kDa, par exemple entre 1 et 12 kDa,
      - un polyamine, tel qu'un chitosan ou une polylysine, ayant notamment des masses moléculaires comprises entre 0,5 et 20 kDa, par exemple entre 1 et 12 kDa.

Par «ester ou amide d'acides gras comprenant de 12 à 24 atomes de carbone et de phosphatidyléthanolamine», on entend un groupe de formule : 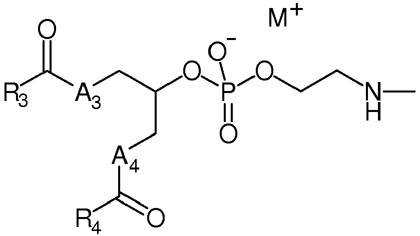 dans laquelle
- R₃ et R₄ représentent indépendamment une chaîne hydrocarbonée linéaire comprenant de 11 à 23 atomes de carbone,
- A₃ et A₄ représentent O ou NH, et
- M représente H ou un cation.

Les groupes cationiques du tensioactif cationique sont typiquement :
- des oniums choisis parmi les groupes ammonium, imidazolium, pyridinium, pyrrolidinium, pipéridinium, phosphonium ou sulfonium, ou
- des complexes métalliques entre un radical d'un groupe organique chélatant mono- ou multi-dentate, par exemple la phénantroline, la pyridine, l'acide éthylène diamine tétraacétique (EDTA), l'acide diéthylène triamine penta acétique (DTPA), les porphyrines, les phtalocyanines, les clorines, les bactériochlorines complexé avec un cation inorganique, tel que Ca²⁺, Al³⁺, Ni⁺, Zn²⁺, Fe²⁺, Fe³⁺ ou Cu²⁺,
les groupes ammonium, notamment-⁺NMe₃, -⁺NHMe₂, -⁺NH₂Me et -⁺NH₃, en particulier -⁺NH₃, étant particulièrement préféré.

Bien sûr, des anions peuvent être associés au(x) groupe(s) cationique(s) pour que la formulation soit électriquement neutre. La nature des anions n'est pas limitée. A titre illustratif, on peut citer les halogénures, notamment le chlorure ou le bromure, ou le trifluoroacétate.

Dans le tensioactif cationique, la nature du groupe de liaison liant le(s) groupe(s) lipophile(s) au(x) groupe(s) hydrophile(s) comprenant au moins un groupe cationique n'est pas limitée. Des exemples de groupes de liaison sont fournis ci-dessous (groupe L).

Dans un mode de réalisation, le tensioactif cationique a la formule (A) suivante :

[(Lipo)ₗ-L-(Hydro)ₕ]ⁿ⁺, (n/m) [A]^{m-} (A)

dans laquelle:
- l et h représentent des nombres entiers indépendamment compris entre 1 et 4,
- n est un nombre entier supérieur ou égal à 1, généralement compris entre 1 et 50,
- Lipo représente un groupe lipophile tel que défini ci-dessus,
- Hydro représente un groupe hydrophile tel que défini ci-dessus comprenant au moins un groupe cationique,
- L représente un groupe de liaison,
- A représente un anion,
- m est un nombre entier représentant la charge de l'anion,
- n est un nombre entier représentant la charge du cation [(Lipo)ₗ-L-(Hydro)ₕ].

Dans la formule (A) susmentionnée, de préférence L est tel que :
a) lorsque l et h représentent 1, L est un groupe de liaison divalent choisi parmi :
   - une liaison simple,
   - un groupe Z choisi parmi -O-, -NH-, -O(OC)-, -(CO)O-, -(CO)NH-, -NH(CO)-, -O-(CO)-O-, -NH-(CO)-O-, -O-(CO)-NH- et -NH-(CO)-NH, -O-PO(OH)-O- ou un radical divalent cyclique de 5 à 6 atomes,
   - un groupe Alk étant un alkylène comprenant de 1 à 6 atomes de carbone, et
   - un groupe Z-Alk, Alk-Z, Alk-Z-Alk ou Z-Alk-Z où Alk et Z sont tels que définis ci-dessus et où les deux groupes Z du groupe Z-Alk-Z sont identiques ou différents,
b) lorsque l'un des groupes l ou h représente 1, et l'autre représente 2, L est un groupe trivalent choisi parmi un groupe phosphate OP-(O-)₃, un groupe dérivé du glycérol de formule -O-CH₂-CH-(O-)CH₂-O- et un radical trivalent cyclique de 5 à 6 atomes,
   - pour les autres valeurs de l et h, L est un radical multivalent cyclique de 5 à 6 atomes.

De manière particulièrement préférée, L est tel que :
a) lorsque l et h représentent 1, L est un groupe de liaison divalent choisi parmi :
   - une liaison simple,
   - un groupe Z choisi parmi -O-, -NH-, -O(OC)-, -(CO)O-, -(CO)NH-, -NH(CO)-, -O-(CO)-O-, -NH-(CO)-O-, -O-(CO)-NH- et -NH-(CO)-NH ou -O-PO(OH)-O-,
b) lorsque l'un des groupes l ou h représente 1, et l'autre représente 2, L est un groupe trivalent choisi parmi un groupe phosphate OP-(O-)₃ et un groupe dérivé du glycérol de formule -O-CH₂-CH-(O-)CH₂-O-.

Dans la formule (A) susmentionnée, l et h représentent de préférence indépendamment 1 ou 2.

Selon une première alternative, le groupe hydrophile du tensioactif cationique est un groupe alkyle linéaire ou ramifié comprenant de 1 à 12 atomes de carbone et interrompu et/ou substitué par au moins un groupe cationique. Comme exemple de tels tensioactifs cationiques, on peut citer:
1) (Lipo)-(CH₂)ₘ₁-NR₃₀R₃₁R₃₂, où Lipo est un groupe lipophile tel que défini ci-dessus, m1 représente 1 ou 2 et R₃₀, R₃₁ et R₃₂ représentent indépendamment H, Me ou -CH₂-CH₂-OH,
2) 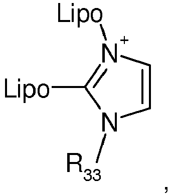 où chaque Lipo est indépendamment un groupe lipophile tel que défini ci-dessus, et R33 représente H, Me ou -CH₂-CH₂-OH,
3) 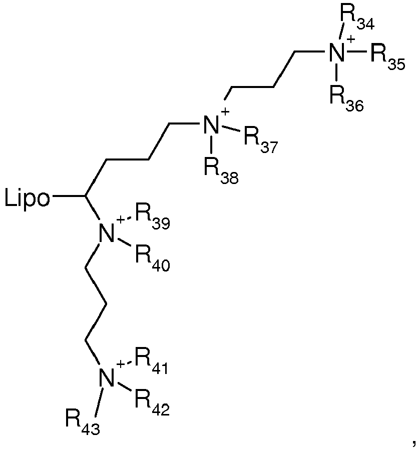 où Lipo est un groupe lipophile tel que défini ci-dessus, et R₃₄, R₃₅, R₃₆, R₃₇, R₃₈, R₃₉, R₄₀, R₄₁, R₄₂ et R₄₃ représentent indépendamment H, Me ou -CH₂-CH₂-OH.

Dans un mode de réalisation, le tensioactif cationique est choisi parmi :
- le *N*[1-(2,3-dioléyloxy) propyl]-*N,N,N*-triméthylammonium (DOTMA),
- le 1,2-dioleyl-3-trimethylamonium-propane (DOTAP),
- le *N*-(2-hydroxyethyl)-*N,N*-dimethyl-2,3-bis(tetradecyloxy-1-propananium) (DMRIE),
- le 1-[2-(oleoyloxy)ethyl]-2-oleyl-3-(2-hydroxyethyl)imidazolinium (DOTIM), et
- le dioctadecylamidoglycylspermine (DOGS) (sous forme protonée),
et est de préférence le 1,2-dioleyl-3-trimethylamonium-propane (DOTAP).

Selon une deuxième alternative, le groupe hydrophile du tensioactif cationique est un groupe polymérique hydrophile comprenant au moins un groupe cationique.

Lorsque le groupe hydrophile du tensioactif cationique est polymérique, le(s) groupe(s) cationique(s) peu(ven)t être un(des) groupe(s) terminal(aux) ou pendant(s). Par exemple :
- lorsque groupe polymérique hydrophile est un poly(oxyde d'éthylène), le(s) groupe(s) cationique(s) est(sont) généralement situé(s) sur un groupe terminal à l'extrémité de la chaîne poly(oxyde d'éthylène).
- lorsque le groupe polymérique hydrophile est du dextrane ou de la cellulose, le(s) groupe(s) cationiques est(sont) généralement situé(s) sur un groupe terminal à l'extrémité de la chaîne polysaccharidique.
- lorsque le groupe polymérique hydrophile est du chitosan, le(s) groupe(s) cationiques est(sont) généralement un(des) groupe(s) pendant(s), en particulier des groupes-NH₃⁺ présents en milieu acide sur le chitosan.

Dans un mode de réalisation, le(s) groupe(s) cationique(s) est(sont) un(des) groupe(s) terminal(aux). En effet, les groupes pendants de tensioactifs anioniques adjacents en surface des gouttelettes de la phase dispersée se repoussent par interactions électrostatiques et, par conséquent, les formulations comprenant des tensioactifs cationiques dont le groupe Hydro comprend des groupes pendants sont généralement moins stables.

Dans un autre mode de réalisation, le(s) groupe(s) cationique(s) est(sont) un(des) groupe(s) pendant(s). Il est avantageusement possible d'utiliser un tensioactif cationique dont le groupe hydrophile comprend plusieurs groupes cationiques pendants, et donc d'obtenir une formulation plus chargée positivement, et qui permettra d'autant mieux la complexation d'espèces négatives comme par exemple les séquences nucléotidiques susceptibles de moduler des mécanismes endogènes d'ARN interférents.

Le groupe polymérique hydrophile préféré est un radical d'un poly(oxyde d'éthylène) comprenant typiquement de 3 à 500 unités oxyde d'éthylène, de préférence de 20 à 200 unités oxyde d'éthylène, et comprenant au moins un groupe cationique.

Ainsi, dans un mode de réalisation, le tensioactif cationique a l'une des formules suivantes : 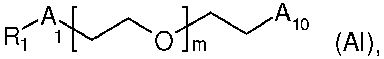 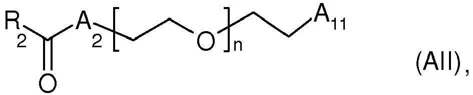 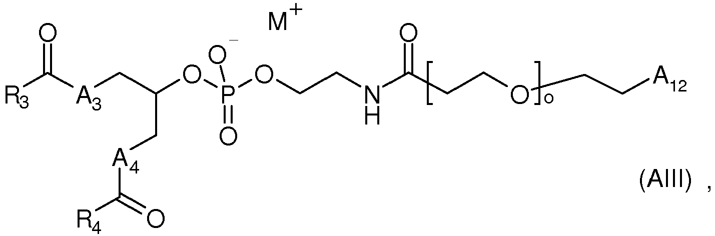 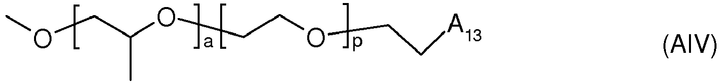 dans lesquelles :
- R₁, R₂, R₃ et R₄ représentent indépendamment une chaîne hydrocarbonée linéaire comprenant de 11 à 23 atomes de carbone,
- A₁, A₂, A₃ et A₄ représentent O ou NH,
- m, n, o et p représentent indépendamment des nombres entiers de 3 à 500, de préférence 20 à 200, et
- a représente un nombre entier de 20 à 120,
- M représente H ou un cation,
- A₁₀, A₁₁, A₁₂ et A₁₃ représentent indépendamment un groupe -⁺NR₂₀R₂₁R₂₂, dans lequel R₂₀, R₂₁ et R₂₂ représentent indépendamment H, Me ou -CH₂-CH₂-OH.

Dans un mode de réalisation, dans la formule (All), A₁₁ représente -⁺NH₃ et le tensioactif cationique a la formule suivante : 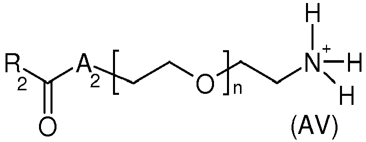 dans laquelle A₂, R₂ et n sont tels que définis ci-dessus. De préférence, dans la formule (All), R₂ représente C₁₇H₃₅.

Sans vouloir être liés à une théorie particulière, la présence du groupe polymérique hydrophile permettrait :
- de stabiliser la formulation, et
- de protéger les molécules localisées à la surface des gouttelettes, plus particulièrement les séquences nucléotidiques susceptibles de moduler des mécanismes endogènes d'ARN interférents, des protéines du milieu dans lequel la formulation est administrée/utilisée, et donc la dégradation des ces molécules, plus particulièrement des séquences nucléotidiques susceptibles de moduler des mécanismes endogènes d'ARN interférents, par ces protéines.

Selon une troisième alternative, la formulation selon l'invention comprend au moins deux tensioactifs cationiques, dont :
a) l'un est choisi parmi :
   - le *N*[1-(2,3-dioléyloxy) propyl]-*N,N,N*-triméthylammonium (DOTMA),
   - le 1,2-dioleyl-3-trimethylamonium-propane (DOTAP),
   - le *N*-(2-hydroxyethyl)-*N,N*-dimethyl-2,3-bis(tetradecyloxy-1-propananium) (DMRIE),
   - le 1-[2-(oleoyloxy)ethyl]-2-oleyl-3-(2-hydroxyethyl)imidazolinium chloride (DOTIM), et
   - le dioctadecylamidoglycylspermine (DOGS),
   et est de préférence le 1,2-dioleyl-3-trimethylamonium-propane (DOTAP), et
b) l'autre est un tensioactif cationique comprenant :
   i) au moins un groupe lipophile choisi parmi :
      - un groupe R ou R-(C=O)-, où R représente une chaîne hydrocarbonée linéaire comprenant de 11 à 23 atomes de carbone,
      - un ester ou un amide d'acides gras comprenant de 12 à 24 atomes de carbone et de phosphatidyléthanolamine, tel que le distéaryl phosphatidyléthanolamine (DSPE), et
      - un poly(oxyde de propylène), et
   ii) un groupe polymérique hydrophile comprenant au moins un groupe cationique, ledit groupe polymérique étant choisi parmi :
      - un poly(oxyde d'éthylène) comprenant typiquement de 3 à 500 unités oxyde d'éthylène, de préférence de 20 à 200 unités oxyde d'éthylène, et comprenant au moins un groupe cationique,
      - un polysaccharide, tel que du dextrane, de la cellulose ou du chitosan,
      - un polyamine, tel qu'un chitosan ou une polylysine,
   et est de préférence un poly(oxyde d'éthylène) comprenant au moins un groupe cationique.

Dans un mode de réalisation, la formulation selon l'invention comprend, à titre de tensioactifs cationiques :
a) du 1,2-dioleyl-3-trimethylamonium-propane, et
b) un tensioactif cationique comprenant :
   i) au moins un groupe lipophile choisi parmi :
      - un groupe R ou R-(C=O)-, où R représente une chaîne hydrocarbonée linéaire comprenant de 11 à 23 atomes de carbone,
      - un ester ou un amide d'acides gras comprenant de 12 à 24 atomes de carbone et de phosphatidyléthanolamine, tel que le distéaryl phosphatidyléthanolamine (DSPE), et
   ii) un poly(oxyde d'éthylène) comprenant typiquement de 3 à 500 unités oxyde d'éthylène, de préférence de 20 à 200 unités oxyde d'éthylène, et comprenant au moins un groupe cationique.

Dans un mode de réalisation, la formulation selon l'invention comprend, à titre de tensioactifs cationiques :
- du 1,2-dioleyl-3-trimethylamonium-propane, et
- un composé de formule (All) telle que définie ci-dessus, notamment de formule (AV).

Le tensioactif cationique se situe dans la couronne des gouttelettes de la formulation. Il se lie par interactions électrostatiques aux molécules chargées négativement, comme par exemple les séquences nucléotidiques susceptibles de moduler des mécanismes endogènes d'ARN interférence et permet de maintenir ces molécules à la surface des gouttelettes.

La formulation comprend de 15 à 70% molaire d'au moins un tensioactif cationique par rapport à l'ensemble (lipide amphiphile / tensioactif cationique / co-tensioactif / éventuel lipide fusogène). En dessous de 15%, la formulation ne comprend pas assez de charges positives et la complexation de la formulation avec les molécules chargées négativement (par exemple les séquences nucléotidiques susceptibles de moduler des mécanismes endogènes d'ARN interférence) est insuffisante. Au-delà de 70%, les formulations ne sont pas stables, et ne peuvent généralement même pas être formulées (la formation de la nanoémulsion n'est pas possible car les gouttelettes coalescent pour former deux phases), et les gouttelettes deviennent généralement toxiques pour les cellules.

Ces proportions sont particulièrement adaptées pour obtenir une complexation efficace des molécules candidates chargées négativement, telles que les séquences nucléotidiques susceptibles de moduler des mécanismes endogènes d'ARN interférents, et donc une bonne délivrance et/ou transfection.

### • Lipide fusogène (« helper lipid » en anglais)

Dans un mode de réalisation, la formulation selon l'invention comprend un lipide fusogène, qui est susceptible de faciliter la libération cytosolique par déstabilisation de la membrane endosomale, dit lipide « helper ». De préférence, ce lipide est le dioléylphosphatidyléthanolamine (DOPE).

Ce lipide permet de favoriser l'échappement endosomal des gouttelettes de la formulation selon l'invention, et donc des molécules candidates, comme par exemple les séquences nucléotidiques susceptibles de moduler des mécanismes endogènes d'interférence par ARN, qu'elles contiennent, et améliore généralement l'efficacité desdites molécules candidates. Par exemple, lorsque la molécule candidate est une séquence nucléotidique susceptible de moduler des mécanismes endogènes d'interférence par ARN, ce lipide améliore l'efficacité d'extinction génique de ladite séquence nucléotidique.

Le lipide susceptible de faciliter la libération cytosolique par déstabilisation de la membrane endosomale se situe dans la couronne des gouttelettes de la formulation.

### • lipide amphiphile

La formulation comprend au moins un lipide amphiphile, qui se situe dans la couronne des gouttelettes de la formulation.

Afin de former une nanoémulsion stable, il est nécessaire d'inclure dans la composition au moins un lipide amphiphile à titre de tensioactif. La nature amphiphile du tensioactif assure la stabilisation des gouttelettes d'huile au sein de la phase continue aqueuse. En dessous de 5% molaire de lipide amphiphile par rapport à l'ensemble (lipide amphiphile / tensioactif cationique / co-tensioactif / éventuel lipide fusogène), les formulations ne sont pas stables, et ne peuvent généralement même pas être formulées (la formation de la nanoémulsion n'est pas possible car les gouttelettes coalescent pour former deux phases).

Généralement, la formulation comprend de 5 à 85% molaire, de préférence de 5 à 75% molaire, en particulier de 5 à 50% molaire et tout particulièrement de 8 à 30% molaire de lipide amphiphile par rapport à l'ensemble (lipide amphiphile / tensioactif cationique / co-tensioactif / éventuel lipide fusogène).

La quantité de lipide amphiphile contribue avantageusement à contrôler la taille de la phase dispersée de la nanoémulsion.

Les lipides amphiphiles comportent une partie hydrophile et une partie lipophile. Ils sont généralement choisis parmi les composés dont la partie lipophile comprend une chaîne saturée ou insaturée, linéaire ou ramifiée, ayant de 8 à 30 atomes de carbone. Ils peuvent être choisis parmi les phospholipides, les cholestérols, les lysolipides, les sphingomyélines, les tocophérols, les glucolipides, stéarylamines, les cardiolipines d'origine naturelle ou synthétique ; les molécules composées d'un acide gras couplé à un groupement hydrophile par une fonction éther ou ester tels que les esters de sorbitan comme par exemple les monooléate et monolaurate de sorbitan vendus sous les dénominations Span^{®} par la société Sigma; les lipides polymérisés ; les lipides conjugués à de courtes chaînes d'oxyde de polyéthylène (PEG) tels que les tensioactifs non-ioniques vendus sous les dénominations commerciales Tween^{®} par la société ICI Americas, Inc. et Triton^{®} par la société Union Carbide Corp.; les esters de sucre tels que les mono- et dilaurate, mono- et di-palmitate, mono- et distéarate de saccharose; lesdits tensioactifs pouvant être utilisés seuls ou en mélanges.

Les phospholipides sont les lipides amphiphiles préférés.

La lécithine est le lipide amphiphile particulièrement préféré.

### • lipide solubilisant

La formulation comprend par ailleurs un lipide solubilisant comprenant au moins un glycéride d'acides gras, qui se situe dans la phase dispersée de la nanoémulsion, plus précisément dans le coeur des gouttelettes. Ce composé a pour mission principale de solubiliser le lipide amphiphile, peu soluble, dans la phase dispersée de la nanoémulsion.

Le lipide solubilisant est un lipide présentant une affinité avec le lipide amphiphile suffisante pour permettre sa solubilisation. De préférence, le lipide solubilisant est solide à température ambiante.

Dans le cas où le lipide amphiphile est un phospholipide, il peut s'agir notamment :
- d'esters d'acides gras et d'alcool gras, comme le cétylpalmitate, ou
- de dérivés du glycérol, et en particulier de glycérides obtenues par estérification de glycérol avec des acides gras.

Le lipide solubilisant utilisé est avantageusement choisi en fonction du lipide amphiphile utilisé. Il présentera généralement une structure chimique proche, afin d'assurer la solubilisation recherchée. Il peut s'agir d'une huile ou d'une cire. De préférence, le lipide solubilisant est solide à température ambiante (20°C), mais liquide à la température du corps (37°C).

Les lipides solubilisants préférés, en particulier pour les phospholipides, sont les esters d'acides gras et d'alcool gras, comme le cétylpalmitate, ou les glycérides d'acides gras, notamment d'acides gras saturés, et en particulier d'acides gras saturés comportant 8 à 18 atomes de carbone, encore préféré 12 à 18 atomes de carbone. Avantageusement, il s'agit d'un mélange de différents glycérides.

De préférence, il s'agit de glycérides d'acides gras saturés comportant au moins 10% en poids d'acides gras en C12, au moins 5% en poids d'acides gras en C14, au moins 5% en poids d'acides gras en C16 et au moins 5% en poids d'acides gras en C18.

De préférence, il s'agit de glycérides d'acides gras saturés comportant 0% à 20% en poids d'acides gras en C8, 0% à 20% en poids d'acides gras en C10, 10% à 70% en poids d'acides gras en C12, 5% à 30% en poids d'acides gras en C14, 5% à 30% en poids d'acides gras en C16 et 5% à 30% en poids d'acides gras en C18.

Particulièrement préférés sont les mélanges de glycérides semi-synthétiques solides à température ambiante vendus sous la dénomination commerciale Suppocire^{®}NC par la société Gattefossé et approuvé pour l'injection chez l'homme. Les Suppocire^{®} de type N sont obtenues par estérification directe d'acides gras et de glycérol. Il s'agit de glycérides hémi-synthétiques d'acides gras saturés de C8 à C18, dont la composition quali-quantitative est indiquée dans le tableau ci-dessous.

Les lipides solubilisants précités permettent d'obtenir une formulation sous forme de nanoémulsion avantageusement stable. Sans vouloir être lié à une théorie particulière, il est supposé que les lipides solubilisants précités permettent d'obtenir des gouttelettes dans la nanoémulsion présentant un coeur amorphe. Le coeur ainsi obtenu présente une viscosité interne élevée sans pour autant présenter de cristallinité. Or, la cristallisation est néfaste pour la stabilité de la nanoémulsion car elle conduit généralement à une agrégation des gouttelettes et/ou à une expulsion des molécules encapsulées à l'extérieur des gouttelettes. Ces propriétés physiques favorisent la stabilité physique de la nanoémulsion.

La quantité de lipide solubilisant peut varier largement en fonction de la nature et de la quantité de lipide amphiphile présent dans la phase dispersée. Généralement, le coeur des gouttelettes (comprenant le lipide solubilisant, l'éventuelle huile, l'éventuel agent d'imagerie, l'éventuel agent thérapeutique s'il est lipophile) comprend de 1 à 100% en poids, de préférence de 5 à 80% en poids et tout particulièrement de 40 à 75% en poids de lipide solubilisant.

**Composition en acides gras du Suppocire^{®} NC de Gattefossé**

| Longueur de chaînes | [% en poids] |
|---|---|
| C8 | 0,1 à 0,9 |
| C10 | 0,1 à 0,9 |
| C12 | 25 à 50 |
| C14 | 10 à 24,9 |
| C16 | 10 à 24,9 |
| C18 | 10 à 24,9 |

### • huile

La phase dispersée peut comporter par ailleurs une ou plusieurs autres huiles, qui se situe(nt) dans le coeur des gouttelettes.

Les huiles utilisées présentent de préférence une balance hydrophile-lipophile (HLB) inférieure à 8 et encore plus préférentiellement comprise entre 3 et 6. Avantageusement, les huiles sont utilisées sans modification chimique ou physique préalablement à la formation de l'émulsion.

Les huiles sont généralement choisies parmi les huiles biocompatibles, et en particulier parmi les huiles d'origine naturelle (végétale ou animale) ou synthétique. Parmi de telles huiles, on peut notamment citer les huiles d'origine naturelle végétale parmi lesquelles figurent notamment les huiles de soja, de lin, de palme, d'arachide, d'olives, de pépin de raisins et de tournesol ; les huiles synthétiques parmi lesquelles figurent notamment les triglycérides, di glycérides et les mono glycérides. Ces huiles peuvent être de premières expressions, raffinées ou inter-estérifiées.

Les huiles préférées sont l'huile de soja et l'huile de lin.

Généralement, si présente, l'huile sera contenue dans le coeur des gouttelettes (comprenant le lipide solubilisant, l'éventuelle huile, l'éventuel agent d'imagerie, l'éventuel agent thérapeutique s'il est lipophile) dans une proportion allant de 1 à 80% en poids, de préférence entre 5 et 50 % en poids et tout particulièrement 10 à 30% en poids.

La phase dispersée peut contenir en outre d'autres additifs tels que des colorants, stabilisants, conservateurs ou d'autres principes actifs, en quantité appropriée.

### • Co-tensioactif

La formulation comprend un co-tensioactif, qui permet de stabiliser la nanoémulsion.

Les co-tensioactifs utilisables dans les formulations utilisées dans l'invention sont généralement des tensioactifs hydrosolubles. Ils comprennent au moins une chaîne poly(oxyde d'éthylène) comprenant au moins 25, notamment au moins 30, de préférence au moins 35, unités d'oxyde d'éthylène. Le nombre d'unités d'oxyde d'éthylène est généralement inférieur à 500.

Des formulations comprenant un co-tensioactif comprenant une chaîne poly(oxyde d'éthylène) comprenant moins 25 unités d'oxyde d'éthylène ne sont en effet pas stables. Généralement, il n'est même pas possible de préparer la nanoémulsion.

Ces nombres d'unités sont en effet particulièrement adaptés pour éviter la fuite des séquences nucléotidiques susceptibles de moduler des mécanismes endogènes d'ARN interférents hors des gouttelettes.

En effet, les inventeurs ont observé qu'une formulation ne comprenant pas un co-tensioactif n'est pas suffisamment stable.

De plus, sans vouloir être liés à une théorie particulière, la présence de la chaîne composée de motifs d'oxyde d'éthylène du co-tensioactif permettrait de protéger les molécules candidates chargées négativement, et plus particulièrement les séquences nucléotidiques susceptibles de moduler des mécanismes endogènes d'ARN interférents, localisées à la surface des gouttelettes des protéines du milieu dans lequel la formulation est administrée/utilisée, et donc la dégradation desdites molécules candidates chargées négativement par ces protéines.

A titre d'exemple de co-tensioactifs, on peut en particulier citer les composés conjugués polyéthylèneglycol/phosphatidyl-éthanolamine (PEG-PE), les éthers d'acide gras et de polyéthylèneglycol tels que les produits vendus sous les dénominations commerciales Brij^{®} (par exemple Brij^{®} 35, 58, 78 ou 98) par la société ICI Americas Inc., les esters d'acide gras et de polyéthylèneglycol tels que les produits vendus sous les dénominations commerciales Myrj^{®} par la société ICI Americas Inc. (par exemple Myrj^{®} 45, 52, 53 ou 59) et les copolymères blocs d'oxyde d'éthylène et d'oxyde de propylène tels que les produits vendus sous les dénominations commerciales Pluronic^{®} par la société BASF AG (par exemple Pluronic^{®} F68, F127, L64, L61, 10R4, 17R2, 17R4, 25R2 ou 25R4) ou les produits vendus sous la dénomination commerciale Synperonic^{®} par la société Unichema Chemie BV (par exemple Synperonic^{®} PE/F68, PE/L61 ou PE/L64).

Ainsi, le co-tensioactif se situe à la fois dans la phase aqueuse continue et dans la phase dispersée. En effet, la partie hydrophobe du co-tensioactif s'insère dans les gouttelettes de la phase dispersée, alors que les chaînes polyalcoxylées sont dans la phase aqueuse continue. Dans la présente demande, les pourcentages massiques de phase dispersée décrits sont calculés en considérant que le co-tensioactif appartient à la phase dispersée.

La formulation comprend de 10% à 55% molaire de co-tensioactif par rapport à l'ensemble (lipide amphiphile / tensioactif cationique / co-tensioactif / éventuel lipide fusogène). En dessous de 10%, les formulations ne sont pas stables, et ne peuvent généralement même pas être formulées (la formation de la nanoémulsion n'est pas possible car les gouttelettes coalescent pour former deux phases). Au-delà de 55%, la complexation de la formulation « premix » avec les molécules candidates chargées négativement, comme par exemple les séquences nucléotidiques, ou avec les étiquettes ADN n'a pas lieu, probablement car les charges positives du tensioactif cationique sont masquées par les chaînes poly(oxyde d'éthylène) du co-tensioactif, et donc plus accessibles pour se lier par liaison électrostatique aux molécules candidates ou aux étiquettes ADN.

### • Phase aqueuse

La phase aqueuse de la nanoémulsion utilisée dans l'invention est de préférence constituée d'eau et/ou d'un tampon tel qu'un tampon phosphate comme par exemple du PBS ("Phosphate Buffer Saline") ou d'une solution saline, notamment de chlorure de sodium.

Selon un mode de réalisation, la phase aqueuse continue comporte également un agent épaississant tel que du glycérol, un saccharide, oligosaccharide ou polysaccharide, une gomme ou encore une protéine, de préférence du glycérol. En effet, l'utilisation d'une phase continue de viscosité plus élevée facilite l'émulsification et permet de ce fait de réduire le temps de sonication.

La phase aqueuse comporte avantageusement de 0 à 50% en poids, de préférence de 1 à 30% en poids et tout particulièrement de 5 à 20% en poids d'agent épaississant.

Bien entendu, la phase aqueuse peut contenir en outre d'autres additifs tels que des colorants, stabilisants et conservateurs en quantité appropriée.

La proportion de phase dispersée et de phase aqueuse est très variable. Cependant, le plus souvent, les nanoémulsions seront préparées avec 1 à 50%, de préférence 5 à 40% et tout particulièrement 10 à 30% en poids de phase dispersée et 50 à 99%, de préférence 60 à 95% et tout particulièrement 70 à 90 % en poids de phase aqueuse.

### • Taille des gouttelettes de la formulation

Les gouttelettes de la formulation définie ci-dessus ont généralement un diamètre compris entre 20 et 200 nm. Ce diamètre peut notamment être mesuré par diffusion quasi-élastique de la lumière sur un appareil ZetaSizer, Malvern.

Il est possible d'obtenir des gouttelettes de taille plus spécifique en adaptant les pourcentages des composants de la nanoémulsion.

Pour une formulation comprenant des gouttelettes de taille comprise entre 20 et 40 nm, on préfèrera utiliser une formulation comprenant au moins 5% molaire de lipide amphiphile et :
- de 25 à 45% molaire de co-tensioactif (en dessous de 25% molaire, la formulation peut présenter des problèmes de stabilité), et/ou
- de 15 à 50% molaire de tensioactif cationique.

Pour une formulation comprenant des gouttelettes de taille comprise entre 40 et 100 nm, on préfèrera utiliser une formulation comprenant au moins 5% molaire de lipide amphiphile et :
- de 45 à 50% molaire de co-tensioactif (en dessous de 45% molaire, la formulation peut présenter des problèmes de stabilité. Au-delà de 50%, l'efficacité en transfection de la formulation est moindre), et/ou
- de 30 à 40% molaire de tensioactif cationique. (en dessous de 30% molaire, l'efficacité en transfection de la formulation est moindre. Au-delà de 40%, la formulation peut présenter des problèmes de stabilité).

Pour une formulation comprenant des gouttelettes de taille comprise entre 130 et 175 nm, on préfèrera utiliser une formulation comprenant au moins 5% molaire de lipide amphiphile et de 15 à 70% molaire d'au moins un tensioactif cationique et :
- de 10 à 25% molaire, notamment de 10 à 15% de co-tensioactif.

Les pourcentages molaires de lipide amphiphile, de tensioactif cationique et de co-tensioactif sont par rapport à l'ensemble (lipide amphiphile / tensioactif cationique / co-tensioactif / éventuel lipide fusogène).

### Traceur

Dans le cadre de la présente invention, on entend par le terme « traceur », une molécule permettant de savoir si la nanoparticule a été intégrée dans une cellule. Dans certains modes de réalisation, ledit traceur est un traceur magnétique, un traceur radioactif ou un fluorophore.

Le traceur magnétique peut être un chélate de gadolinium ou un nanocristal magnétique, comme par exemple un nanocristal de fer, d'oxyde de manganèse ou de fer-platine (Fe-Pt).

Le traceur radioactif peut être un composé comprenant un radionucléide, tel que par exemple ¹²³I, ¹⁸F, ¹¹C, ou un chélate de ^{99m}Tc ou ¹¹¹In, ou un chélate de cations métalliques ⁶⁸Ga, ⁶⁴Cu.

Le fluorophore est de préférence un fluorophore lipophile. De préférence, les fluorophores utilisés absorbent et émettent dans le visible ou le proche infrarouge. Le fluorophore sélectionné est adapté au type d'application du procédé : in-vivo ou in-vitro. Dans le cas des applications in-vivo, on peut avoir recours à l'imagerie de fluorescence in-vivo. Pour cette imagerie, non invasive, les fluorophores préférés absorbent et émettent dans le proche infrarouge. En effet, pour que la lumière d'excitation et la lumière émise par le fluorophore puissent mieux traverser les tissus, il convient d'utiliser des fluorophores absorbant et émettant dans le proche infrarouge, c'est-à-dire à une longueur d'onde comprise entre 640 et 900 nm. Lors des applications in-vitro, pour lesquelles on met en oeuvre des modalités d'imagerie telle la microscopie de fluorescence ou la cytométrie de type FACS (Fluorescence Activating Cell Sorting), on met généralement en oeuvre des fluorophores excités par des longueurs d'onde visibles, typiquement comprises entre 450 nm et 650 nm. Il s'agit notamment de cyanines hydrophiles ou du FITC (Fluorescein isothiocyanate). Ces fluorophores peuvent être encapsulés dans le coeur lipidique des nanoparticules ou, lorsqu'ils sont hydrophiles, être localisés à leur surface.

A titre de fluorophore lipophile on peut par exemple citer les composés décrits dans le chapitre 13 ("Probes for Lipids and Membranes") du catalogue InVitrogen (The Molecular Probes® Handbook, a guide to fluorescent probes and labeling technologies, 11^{ème} Edition, 2010). De façon plus précise, on peut notamment citer, à titre de fluorophore, le vert d'indocyanine (ICG), les analogues d'acides gras et les phospholipides fonctionnalisés par un groupement fluorescent tels que les produits fluorescents vendus sous les dénominations commerciales Bodipy (R) tels que le Bodipy (R) 665/676 (Ex/Em.) ; les dérivés lipophiles de carbocyanines telles que le perchlorate de 1,1'--dioctadécyl-3,3,3',3'-tétraméthylindodicarbocyanine (DiD), par exemple vendu sous la référence D-307, le perchlorate de 3,3'-dihexadecyloxacarbocyanine (DiO), par exemple vendu sous la référence D1125, le perchlorate de 1,1'-dihexadecyl-3,3,3',3'-tetramethylindocarbocyanine (DiI), par exemple vendu sous la référence D384; les sondes fluorescentes dérivées de sphingolipides, de stéroïdes ou de lipopolysaccharides tels que les produits vendus sous les dénominations commerciales BODIPY ® TR céramides, BODIPY ® FL C5-lactosylcéramide, BODIPY ® FL C5-ganglioside, BODIPY ® FL cérébrosides ; les dérivés amphiphiles de cyanines, de rhodamines, de fluorescéines ou de coumarines tels que l'octadécyl rhodamine B, l'octadécyl ester de fluorescéine et la 4-heptadécyl-7-hydroxycoumarine ; et le diphénylhexatriène (DPH) et ses dérivés ; l'ensemble de ces produits étant vendus par la société Invitrogen.

Selon une forme de réalisation préférée de l'invention, le fluorophore est le vert d'indocyanine, le perchlorate de 1,1'--dioctadécyl-3,3,3',3'-tétraméthylindodicarbocyanine, le perchlorate de 3,3'-dihexadecyloxacarbocyanine, ou le perchlorate de 1,1'-dihexadecyl-3,3,3',3'-tetramethylindocarbocyanine.

### Molécule candidate

Dans le cadre de la présente invention, on entend par le terme « molécule candidate », toute molécule capable d'engendrer un changement phénotypique dans une cellule et/ou un tissu et/ou un organe.

Dans certains mode de réalisation, ladite molécule candidate est une molécule neutre ou une molécule chargée négativement ou positivement. De préférence, ladite molécule candidate est chargée négativement.

A titre d'exemples, lorsque la nanoparticule telle que définie ci-dessus est une nanoparticule anionique, ladite molécule candidate est chargée positivement, ou lorsque ladite nanoparticule telle que définie ci-dessus est une nanoparticule cationique, ladite molécule candidate est chargée négativement.

Dans certains modes de réalisation, ladite molécule candidate est une molécule chimique. De préférence, ladite molécule chimique est un principe actif pharmaceutique, une biomolécule, un agent utile pour des thérapies physiques, ou un agent radioactif. De préférence, ladite molécule chimique est un agent thérapeutique.

Parmi les principes actifs pharmaceutiques intéressants comme agents thérapeutiques, on peut citer en particulier les agents utilisés dans le traitement du SIDA, les agents utilisés dans le traitement des maladies cardiaques, les analgésiques, les anesthésiques, les anorexigènes, les anthelmintiques, les antiallergiques, les antiangineux, les antiarythmisants, les anticholinergiques, les anticoagulants, les antidépresseurs, les antidiabétiques, les antidiurétiques, les antiémétiques, les anticonvulsivants, les antifongiques, les antihistaminiques, les antihypertenseurs, les anti-inflammatoires, les anti-migraineux, les antimuscariniques, les antimycobactériens, les anticancéreux y compris les antiparkinsoniens, les antithyroïdiens, les antiviraux, les astringents, les agents bloquants, les produits sanguins, les substituts sanguins, les agents inotropes cardiaques, les agents cardiovasculaires, les agents du système nerveux central, les chélateurs, les agents de chimiothérapie, les facteurs de croissance hématopoïétiques, les corticostéroïdes, les antitussifs, les agents dermatologiques, les diurétiques, les dopaminergiques, les inhibiteurs de l'élastase, les agents endocrines, les alkaloïdes de l'ergot, les expectorants, les agents gastro-intestinaux, les agents génito-urinaires, le facteur de déclenchement de l'hormone de croissance, les hormones de croissance, les agents hématologiques, les agents hématopoïétiques, les hemostatiques, les hormones, les immunosuppresseurs, les interleukines, les analogues d'interleukines, les agents de régulation des lipides, la gonadolibérine, les myorelaxants, les antagonistes narcotiques, les nutriments, les agents nutritifs, les thérapies oncologiques, les nitrates organiques, les vagomimétiques, les prostaglandines, les antibiotiques, les agents rénaux, les agents respiratoires, les sédatifs, les hormones sexuelles, les stimulants, les sympathomimétiques, les anti-infectieux systémiques, le tacrolimus, les agents thrombolytiques, les agents thyroïdiens, les traitements pour les troubles de l'attention, les vasodilatateurs, les xanthines, les agents diminuant le cholestérol. Particulièrement visés sont les anticancéreux tels que le taxol (paclitaxel), la doxorubicine et le cisplatine.

Parmi les biomolécules, on peut citer les protéines, les peptides, les anticorps, les saccharides, et les acides nucléiques.

Parmi les agents utiles pour des thérapies physiques, on peut citer les composés utiles pour la thermothérapie, les composés relarguant de l'oxygène singulet après excitation par une lumière utile pour la photothérapie (c'est-à-dire des photo-sensibilisateurs) et les agents radioactifs.

Parmi les agents radioactifs, on peut citer notamment les isotopes radioactifs.

Parmi les photo-sensibilisateurs, on peut citer notamment ceux appartenant à la classe des tétrapyrroles comme les porphyrines, les bactériochlorines, les phtalocyanines, les chlorines, les purpurines, les porphycènes, les phéophorbides, ou encore ceux appartenant à la classe des texaphyrines ou des hypericines. Parmi les photo-sensibilisateurs de première génération, on peut mentionner l'hémato-porphyrine et un mélange de dérivés d'hémato-porphyrine (HpD) (vendu sous la marque commerciale Photofrin^{®} par Axcan Pharma). Parmi les photo-sensibilisateurs de seconde génération, on peut mentionner le méta-tetra-hydroxyphenyl chlorine (mTHPC ; nom commercial Foscan^{®}, Biolitec AG) et le dérivé monoacide du cycle A de la benzoporphyrine (BPD-MA vendu sous la marque commerciale Visudyne^{®} par QLT et Novartis Opthalmics). Les formulations des photo-sensibilisateurs de seconde génération qui associent à ces photo-sensibilisateurs une molécule (lipide, peptide, sucre etc..) qualifiée de transporteur qui permet leur acheminement sélectif au niveau du tissu tumoral sont appelées photo-sensibilisateurs de troisième génération.

Dans un mode de réalisation, ladite molécule candidate chargée négativement est un acide nucléique. De préférence, ledit acide nucléique comprend ou consiste en une séquence nucléotidique susceptible de moduler des mécanismes d'interférence par ARN. Dans certains modes de réalisation, ladite séquence nucléotidique comprend des nucléotides modifiés, tel que l'inosine.

Par « séquence nucléotidique susceptible de moduler des mécanismes d'interférence par ARN », on entend un acide nucléique antisens qui se lie à une cible ARN via des interactions ARN-ARN, ARN-ADN ou protéine-ARN (Egholm et al., 1993, Nature, 365: 566) et altère l'activité de la cible ARN (pour revue, voir Stein et Cheng, 1993, Science, 261, 1004 et Woolf et al., U.S. Pat. No. 5,849,902). Généralement, une séquence contiguë de l'acide nucléique antisens est complémentaire d'une séquence cible. Toutefois, dans certains modes de réalisation, l'acide nucléique antisens peut se lier à un substrat de sorte que le substrat forme une boucle ou une structure en épingle à cheveux, et/ou l'acide nucléique antisens peut se replier de sorte à former une boucle ou une structure en épingle à cheveux. Ainsi, (i) l'acide nucléique antisens peut être complémentaire de 2, 3, 4, 5, 6, 7, 8, 9, 10, ou plus, séquences substrat non-contigues, et/ou (ii) 2, 3, 4, 5, 6, 7, 8, 9, 10, ou plus, portions de séquence non contigue de l'acide nucléique antisens peuvent être complémentaires de la séquence cible (pour exemple, voir Crooke, 2000, Methods Enzymol., 313: 3-45).

L'acide nucléique antisens entre dans une voie cellulaire qui est communément appelée voie de l'interférence par ARN (ARNi) (« RNAi » en anglais). Le terme « interférence par ARN » se réfère à la dégradation sélective intracellulaire de l'ARN, aussi connue sous le nom de « gene silencing ». L'ARNi inclut aussi la répression de la traduction par les petit ARN interférents (siARN) (« siRNA » en anglais). L'ARNi peut être par exemple initiée par l'introduction d'ARN long double-brin (dsRNA) ou d'un siARN.

Dans certains modes de réalisation, ladite séquence nucléotidique susceptible de moduler des mécanismes d'interférence par ARN est :
- un petit ARN interférent (siARN) (« short-interfering RNA » ou « small interfering RNA » siRNA en anglais),
- un acide nucléique bloqué (« Locked Nucleic Acid » LNA en anglais), ou
- un microARN (miARN) (« MicroRNA » ou « miRNA » en anglais), ou
- un ARN long double brin (ARNdb) (« Long double-stranded RNA » ou « dsRNA » en anglais).

Un « petit ARN interférent » ou « siARN » est un duplex ARN de nucléotides qui est destiné à cibler un gène d'intérêt. Un duplex ARN se réfère à une structure formée par l'appariement complémentaire entre deux régions de la molécule d'ARN. Dans certains modes de réalisation, la longueur du duplex du siARN est comprise entre 15 nucléotides et 50 nucléotides, de préférence entre 20 nucléotides et 35 nucléotides, de préférence encore entre 21 nucléotides et 29 nucléotides. Dans certains mode de réalisation, le duplex peut être long d'au moins 15, 16, 17, 18, 19, 20, 21, 22, 23, 24, 25, 26, 27, 28, 29, 30, 31, 32, 33, 34, 35, 40, 45, 50 nucléotides. Dans certains mode de réalisation, le duplex peut être long d'au plus 45, 40, 35, 34, 33, 32, 31, 30, 29, 28, 27, 26, 25, 24, 23, 22, 21, 20, 19, 18, 17, 16, 15 nucléotides. La portion duplex du siARN peut faire partie d'une structure en épingle à cheveux. En plus de la portion en duplex, la structure en épingle à cheveux peut contenir une boucle entre les deux séquences formant le duplex. La boucle peut varier en longueur. Dans certains modes de réalisation, la longueur de la boucle est de 5, 6, 7, 8, 9, 10, 11, 12, ou 13 nucléotides. Deux déoxythymidines sont généralement ajoutées en partie 3' de chacun des ses brins pour en augmenter la stabilité. Ainsi, un siARN dont des deoxythymidine ont été greffés à ses parties 3' ne sort pas de la définition d'un siARN selon la présente demande. Un siARN permet de diminuer l'expression d'une protéine cible par interférence avec l'ARN messager codant pour cette protéine.

Le terme « ARN long double brin » (dsARN) (« Long double-stranded RNA » ou « dsRNA » en anglais) se réfère à un oligoribonucléotide ou un polyribonucléotide, modifié ou non modifié, ainsi qu'en ses fragments ou portions, d'origine génomique ou synthétique ou dérivé de l'expression d'un vecteur d'expression, qui peut être partiellement ou totalement double brin et dont les extrémités peuvent être franches (« blunt ended » en anglais) ou contenir des extrémités sortantes en 5' et en 3', et qui peut aussi avoir une forme en épingle à cheveux. Dans certains modes de réalisation, le dsARN a une taille comprise entre 250 pb à 2000 pb, de préférence entre 300 pb et 1000 pb. Dans certains modes de réalisation, le dsARN a une taille d'au moins 150, 160, 170, 180, 190, 200, 210, 220, 230, 240, 250, 260, 270, 280, 290, 300, 310, 320, 330, 340, 350, 400, 450, 500, 550, 600, 650, 700, 750, 800, 850, 900, 950, 1000, 1500 pb. Dans certains modes de réalisation, le dsARN a une taille d'au plus 3000, 2500, 2000, 1500, 1000, 950, 900, 850, 800, 750,700, 650, 600, 550, 500, 450, 400, 350, 300 pb.

Dans un mode de réalisation, ladite séquence nucléotidique est un acide nucléique bloqué.

Un acide nucléique bloqué est une séquence nucléotidique d'ARN et/ou d'ADN mono brin dont au moins un des acides nucléiques contient un pont méthylène entre l'hydroxyle en position 2 et l'atome de carbone 4 du ribose. C'est une séquence nucléotidique synthétique. Un acide nucléique bloqué est un inhibiteur de microARN et permet de réguler l'expression d'une ou plusieurs protéines cibles dont les ARNm étaient en interférence avec les séquences ARN issues du dit microARN. La régulation est le plus souvent une levée d'inhibition d'expression des protéines.

Dans un mode de réalisation, ladite séquence nucléotidique est un microARN.

Un microARN est une séquence nucléotidique d'ARN simple brin (de l'ordre de la centaine de bases). C'est une séquence nucléotidique synthétique.

Un microARN permet de réguler l'expression d'une ou plusieurs protéines cibles par interférence avec un ou plusieurs ARNm codant respectivement pour ces protéines. La régulation est le plus souvent une inhibition de l'expression des protéines.

### Etiquette ADN unique

Par « étiquette ADN unique », on entend une séquence nucléotidique double brin ou simple brin spécifique d'une molécule candidate.

Dans certains modes de réalisation, ladite étiquette ADN unique consiste en une séquence d'ADN simple brin d'au moins 50, 55, 60, 65, 70, 75, 80, 85, 90, 95, 100 nucléotides, ou d'au plus 100, 95, 90, 85, 80, 75, 70, 65, 60, 55, 50 nucléotides. Dans certains modes de réalisation, ladite étiquette ADN unique consiste en une séquence d'ADN double brin d'au moins 50, 55, 60, 65, 70, 75, 80, 85, 90, 95, 100 paires de bases (pb), ou d'au plus 100, 95, 90, 85, 80, 75, 70, 65, 60, 55, 50 pb.

De préférence, dans la bibliothèque de nanoparticules selon l'invention ou dans les méthodes de criblage selon l'invention, ladite étiquette ADN unique consiste en une séquence d'ADN, de préférence double brin, d'au moins 50 nucléotides ou pb consistant en, sur un brin et dans le sens 5'-3' :
- une première séquence d'au moins 20 nucléotides commune à toutes les étiquettes ADN unique de la bibliothèque,
- une séquence unique d'au moins 10 nucléotides propre à la molécule candidate telle que définie ci-dessus,
- une deuxième séquence d'au moins 20 nucléotides commune à toutes les étiquettes ADN unique de la bibliothèque.

Lesdites première et deuxième séquences sont notamment utiles pour amplifier et/ou séquencer ladite étiquette ADN unique. A cette fin, il est préférable que lesdites premières ou deuxièmes séquences aient une séquence nucléotidique qui n'existe pas dans le génome de la cellule, du tissu ou de l'organisme d'où provient la cellule mise en contact avec la bibliothèque de nanoparticules. Par exemple, (i) lorsque la cellule mise en contact avec la bibliothèque de nanoparticules est une cellule humaine, lesdites première et deuxième séquences ne font pas partie du génome humain ; (ii) lorsque la cellule mise en contact avec la bibliothèque de nanoparticules est une cellule d'un animal, telle qu'une cellule de rongeur (rat, souris), lesdites première et deuxième séquences ne font pas partie du génome dudit animal ; (iii) lorsque la cellule mise en contact avec la bibliothèque de nanoparticules est une cellule d'une plante, lesdites première et deuxième séquences ne font pas partie du génome de ladite plante. De préférence, lesdites premières ou deuxièmes séquences comprennent au moins 1, 2, 3, 4, 5, 6, ou plus de, nucléotides modifiés. Par exemple, lesdites premières ou deuxièmes séquences comprennent au moins 1, 2, 3, 4, 5, 6, ou plus de, inosine.

Dans certains modes de réalisation, lesdites première et deuxième séquences sont identiques ou différentes, de préférence lesdites première et deuxième séquences sont différentes. Cela permet d'utiliser un unique couple d'amorces pour l'ensemble des étiquettes, lors de l'étape d'amplification. Dans certains modes de réalisation, lesdites première et deuxième séquences ont une longueur d'au moins 20, 25, 30, 35, 40, 45, 50 nucléotides, ou d'au plus 50, 45, 40, 35, 30, 25, 20 nucléotides.

Par « séquence unique d'au moins 10 nucléotides propre à la molécule candidate », on entend une séquence associée *in silico* à une molécule candidate déterminée. Ainsi, dans une bibliothèque de nanoparticules, chaque molécule candidate est associée à une séquence unique d'au moins 10 nucléotides et peut être identifiée grâce à cette séquence unique contenue dans l'étiquette ADN unique. Dans certains modes de réalisation, ladite séquence unique propre à la molécule candidate a une longueur d'au moins 10, 11, 12, 13, 14, 15, 16, 17, 18, 19, 20, 21, 22, 23, 24, 25, 30, 35, 40, 45, 50 nucléotides, ou d'au plus 50, 45, 40, 35, 30, 25, 24, 23, 22, 21, 20, 19, 18, 17, 16, 15, 14, 13, 12, 11, 10 nucléotides.

Dans certains modes de réalisation, ladite séquence unique a une séquence nucléotidique qui n'existe pas dans le génome de la cellule, du tissu ou de l'organisme d'où provient la cellule mise en contact avec la bibliothèque de nanoparticules. Par exemple, (i) lorsque la cellule mise en contact avec la bibliothèque de nanoparticules est une cellule humaine, ladite séquence unique ne fait pas partie du génome humain ; (ii) lorsque la cellule mise en contact avec la bibliothèque de nanoparticules est une cellule d'un animal, telle qu'une cellule de rongeur (rat, souris), ladite séquence unique ne fait pas partie du génome dudit animal ; (iii) lorsque la cellule mise en contact avec la bibliothèque de nanoparticules est une cellule d'une plante, ladite séquence unique ne fait partie du génome de ladite plante. De préférence, ladite séquence unique comprend au moins 1, 2, 3, 4, 5, 6, ou plus de, nucléotides modifiés. Par exemple, ladite séquence unique comprend au moins 1, 2, 3, 4, 5, 6, ou plus de, inosine.

La synthèse desdites étiquettes ADN unique peut être réalisée par des procédés connus de l'homme du métier.

### Ligand biologique de ciblage d'une cellule et/ou d'un organe

Le ligand biologique de ciblage peut être par exemple un anticorps, un peptide, un saccharide, un aptamère, un oligonucléotide ou un composé comme l'acide folique.

Selon un mode réalisation, ledit ligand biologique de ciblage est greffé en surface avec un composé amphiphile, notamment avec le co-tensioactif, de la gouttelette de la formulation sous forme d'une nanoémulsion comprenant une phase aqueuse continue et au moins une phase dispersée telle que définie ci-dessus. La nanoémulsion comprend alors un co-tensioactif greffé. Dans ce cas, le co-tensioactif joue le rôle d'un espaceur permettant d'accommoder ligand biologique de ciblage en surface. Par exemple, lorsque le ligand biologique est un peptide comprenant une ou plusieurs cystéines, le greffage à la chaîne oxyde d'alkylène du tensioactif peut être assuré par couplage thiol maléimide.

### Localisation des composants des nanoparticules

### • Lorsque la nanoparticule est inorganique

Lorsque la nanoparticule est inorganique, e.g. lorsque la nanoparticule est une nanoparticule d'or, ou une nanoparticule magnétique ou un nanocristal de matériau semi-conducteur, la molécule candidate, le traceur et l'étiquette ADN unique tels que définis ci-dessus sont localisées à la surface de ladite nanoparticule inorganique. De préférence, la molécule candidate, le traceur et l'étiquette ADN unique tels que définis ci-dessus sont complexés de façon non covalente à la surface de ladite nanoparticule inorganique.

### • Lorsque la nanoparticule est organique

Lorsque la nanoparticule est organique, e.g. lorsque la nanoparticule est un vecteur cationique, la molécule candidate, le traceur et/ou l'étiquette ADN unique tels que définis ci-dessus sont localisés à la surface de ladite nanoparticule organique et/ou sont encapsulés dans ladite nanoparticule organique, selon la nature de la molécule candidate, du traceur et de l'étiquette ADN unique.

Dans certains modes de réalisation, (i) lorsque ladite nanoparticule est une gouttelette d'une formulation sous forme d'une nanoémulsion comprenant une phase aqueuse continue et au moins une phase dispersée telle que définie ci-dessus et (ii) lorsque ladite molécule candidate est chargée négativement :
- ladite molécule candidate chargée négativement et ladite étiquette ADN unique sont maintenues à la surface des gouttelettes de la phase dispersée de la formulation grâce aux interactions électrostatiques avec le tensioactif cationique, i.e. elles sont donc localisées à la surface des gouttelettes, au niveau de la couronne des gouttelettes, du côté hydrophile de la couronne,
- le traceur tel que défini ci-dessus est localisé dans le coeur des gouttelettes.

En conséquence, dans un mode de réalisation préféré, les gouttelettes de la formulation selon l'invention s'organisent sous forme de coeur - couronne, où :
- le coeur comprend :
   - le lipide solubilisant,
   - l'éventuelle huile,
   - le traceur,
- la couronne comprend :
   - le lipide amphiphile,
   - le tensioactif cationique,
   - le co-tensioactif (éventuellement greffé avec une molécule d'intérêt),
   - la séquence nucléotidique susceptible de moduler des mécanismes endogènes d'ARN interférence,
   - l'étiquette ADN unique,
   - l'éventuel lipide fusogène,
   - l'éventuel tensioactif de formule (I),
   - l'éventuel ligand biologique de ciblage.

Outre l'éventuelle liaison des siARN à des deoxythymidine mentionnées ci-dessus, lesdites séquences nucléotidiques ne sont pas modifiées chimiquement et elles ne sont pas dénaturées. En particulier, lesdites séquences nucléotidiques ne sont pas liées de façon covalente aux nanoparticules. Notamment, lesdites séquences nucléotidiques ne sont liées de façon covalente ni au co-tensioactif, ni au lipide amphiphile, ni à l'éventuel agent d'imagerie. En effet, lesdites séquences nucléotidiques sont uniquement liées aux gouttelettes de la nanoémulsion par interactions électrostatiques avec les tensioactifs cationiques. Ceci est très avantageux car lesdites séquences nucléotidiques, une fois libérées dans leur site d'action, ne sont pas dénaturées et peuvent jouer le rôle attendu. De plus, il n'est pas nécessaire de préparer des dérivés de séquences nucléotidiques, ce qui est coûteux. Les séquences nucléotidiques disponibles dans le commerce peuvent donc être complexées aux gouttelettes sans modification préalable.

Dans certains modes de réalisation, (i) lorsque ladite nanoparticule est une gouttelette d'une formulation sous forme d'une nanoémulsion comprenant une phase aqueuse continue et au moins une phase dispersée telle que définie ci-dessus et (ii) lorsque ladite molécule candidate est une molécule chimique, les gouttelettes de la formulation selon l'invention s'organisent sous forme de coeur - couronne, où :
- le coeur comprend :
   - le lipide solubilisant,
   - l'éventuelle huile,
   - le traceur,
   - la molécule chimique si elle est lipophile,
- la couronne comprend :
   - le lipide amphiphile,
   - le tensioactif cationique,
   - le co-tensioactif (éventuellement greffé avec une molécule d'intérêt),
   - la molécule chimique si elle est amphiphile,
   - l'étiquette ADN unique,
   - l'éventuel lipide fusogène,
   - l'éventuel tensioactif de formule (I),
   - l'éventuel ligand biologique de ciblage.

Ces modes de réalisation présentent en outre les avantages supplémentaires suivants, lorsque la molécule candidate est localisée à la surface de chaque nanoparticule :
- il suffit de préparer une seule et même émulsion pour former les nanoparticules, qui sont ensuite greffées avec les différentes molécules candidates, pour constituer la bibliothèque de nanoparticules ;
- lorsque la nanoparticule est une gouttelette dans laquelle le fluorophore (et/ou l'étiquette ADN) est encapsulé, ceci permet de limiter le risque d'interaction entre le fluorophore (et/ou l'étiquette ADN) et la molécule candidate, cette dernière étant localisée à la surface de la gouttelette.

### Bibliothèque de nanoparticules

Dans certains modes de réalisation, la bibliothèque de nanoparticules comprend ou consiste en au moins 2, 10, 50, 100, 250, 500, 1000, 2500, 5000, 7500, 10000, 12500, 15000, 20000, 30000, 50000, 75000, 100000, ou plus nanoparticules telles que définies ci-dessus. Dans certains modes de réalisation ladite bibliothèque comprend ou consiste en un nombre de nanoparticules compris entre 2 et 100000, de préférence entre 500 et 50000, de préférence encore entre 1000 et 15000.

Dans certains modes de réalisation, ladite bibliothèque de nanoparticules comprend ou consiste en un nombre de nanoparticules au moins égal au nombre de molécules candidates présentes dans une collection de molécules disponible commercialement, ou au moins égal à un nombre de molécules candidates apparentées fonctionnellement (e.g. des molécules candidates ayant la même cible, par exemple des inhibiteurs d'une même classe d'enzyme, d'une même voie métabolique, ou d'un même gène, des molécules candidates apparentées fonctionnellement ont le même mécanisme d'action sur une voie métabolique ou sur une voie de signalisation), c'est-à-dire qu'il existe au moins une copie de chaque type de nanoparticule, le type étant défini par la combinaison de la molécule candidate, le traceur et l'étiquette ADN unique.

Dans certains modes de réalisation, ladite bibliothèque de nanoparticules comprend ou consiste en au moins 1, 2, 10, 50, 100, 250, 500, 1000, 2500, 5000, 7500, 10000, 20000, 50000, 75000, 100000 type(s) de nanoparticules, chaque type de nanoparticules étant présent en un nombre de copies au moins égal à 10, 50, 100, 500, 1000, 2000, 5000, 7500, 10000, 15000, 20000 nanoparticules.

A titre d'exemples non limitatifs de collection de molécules candidates, on peut notamment citer la collection de 1292 siARN de Qiagen ciblant 646 kinases (Human Kinase siRNA set V1.0 ; Ref 1027091), ou la collection de 2375 siARN de Qiagen ciblant 1183 gènes impliqués dans les cancers (Human Cancer siRNA set V2.0), ou la collection de 278 siARN ciblant 139 gènes impliqués dans les cancers (Human Cancer siRNA set V1.0; Ref 1022171), ou la collection de 91800 siARN de Qiagen ciblant 22950 gènes humains (Human Genome Wide siRNA set), ou la collection de 982 LNA d'Exiqon ciblant tous les miARN humains connus (miRCURY LNA Human microRNA Inhibitor Library; Ref 190102-2).

A titre d'exemple non limitatif, ladite bibliothèque de nanoparticules comprend ou consiste en 1292 nanoparticules, chaque nanoparticule comprenant l'un des siARN de la collection de 1292 siARN de Qiagen ciblant 646 kinases, un traceur et une étiquette ADN unique propre audit siARN; ou ladite bibliothèque de nanoparticules comprend ou consiste en 2375 nanoparticules, chaque nanoparticule comprenant l'un des siARN de la collection de 2375 siARN de Qiagen ciblant 1183 gènes impliqués dans les cancers, un traceur et une étiquette ADN unique propre audit siARN; ou ladite bibliothèque de nanoparticules comprend ou consiste en 278 nanoparticules, chaque nanoparticule comprenant l'un des siARN de la collection de 278 siARN ciblant 139 gènes impliqués dans les cancers, un traceur et une étiquette ADN unique propre audit siARN ; ou ladite bibliothèque de nanoparticules comprend ou consiste en 91800 nanoparticules, chaque nanoparticule comprenant l'un des siARN de la collection de 91800 siARN de Qiagen ciblant 22950 gènes humains, un traceur et une étiquette ADN unique propre audit siARN ; ou ladite bibliothèque de nanoparticules comprend ou consiste en 982 nanoparticules, chaque nanoparticule comprenant l'un des LNA de la collection de 982 LNA d'Exiqon ciblant tous les miARN humains connus, un traceur et une étiquette ADN unique propre audit LNA.

Ladite bibliothèque de nanoparticules peut également comprendre ou consister en au moins 1, 2, 3, 4, 5, 10, 15, 20, 50, 100, 500, 1000, 2500, 5000 familles de nanoparticules, chaque famille comprenant au moins 2, 3, 4, 5, 10, 15, 20, 25, 50, 100, 200 types de nanoparticules comprenant un même traceur et les molécules candidates étant apparentées fonctionnellement (e.g. des molécules candidates ayant la même cible, par exemple des inhibiteurs d'une même classe d'enzyme, d'une même voie métabolique, ou d'un même gène, des molécules candidates apparentées fonctionnellement ont le même mécanisme d'action sur une voie métabolique ou sur une voie de signalisation) ou étant présentes dans une collection de molécules disponible commercialement, comme par exemple l'une des collections citées ci-dessus.

Par exemple, ladite bibliothèque de nanoparticules peut comprendre ou consister en 2 familles de nanoparticules, la première famille de nanoparticules comprenant ou consistant en 1292 types de nanoparticules, chaque type de nanoparticules comprenant l'un des siARN de la collection de 1292 siARN de Qiagen ciblant 646 kinases, un traceur et une étiquette ADN unique propre audit siARN; et la seconde famille de nanoparticules comprenant 2375 types de nanoparticules, chaque type de nanoparticules comprenant l'un des siARN de la collection de 2375 siARN de Qiagen ciblant 1183 gènes impliqués dans les cancers, un traceur et une étiquette ADN unique propre audit siARN, étant entendu que les traceurs des première et seconde familles de nanoparticules sont différents.

Selon un autre exemple, ladite bibliothèque de nanoparticules peut comprendre ou consister en 5 familles de nanoparticules, la première famille de nanoparticules comprenant ou consistant en 1292 types de nanoparticules, chaque type de nanoparticules comprenant l'un des siARN de la collection de 1292 siARN de Qiagen ciblant 646 kinases, un traceur et une étiquette ADN unique propre audit siARN; la deuxième famille de nanoparticules comprenant ou consistant en 2375 types de nanoparticules, chaque type de nanoparticules comprenant l'un des siARN de la collection de 2375 siARN de Qiagen ciblant 1183 gènes impliqués dans les cancers, un traceur et une étiquette ADN unique propre audit siARN ; la troisième famille de nanoparticules comprenant ou consistant en 278 types de nanoparticules, chaque types de nanoparticules comprenant l'un des siARN de la collection de 278 siARN de Qiagen 139 gènes impliqués dans les cancers, un traceur et une étiquette ADN unique propre audit siARN ; la quatrième famille de nanoparticules comprenant ou consistant en 91800 types de nanoparticules, chaque type de nanoparticules comprenant l'un des siARN de la collection de 91800 siARN de Qiagen ciblant 22950 gènes humains, un traceur et une étiquette ADN unique propre audit siARN, la cinquième famille de nanoparticules comprenant ou consistant en 982 types de nanoparticules, chaque types de nanoparticules comprenant l'un des LNA de la collection de 982 LNA d'Exiqon ciblant tous les miARN humains connus, un traceur et une étiquette ADN unique propre audit LNA, étant entendu que les traceurs des première, deuxième, troisième, quatrième et cinquième familles de nanoparticules sont différents.

### Cellules

Les cellules utilisées dans les méthodes de criblage selon l'invention peuvent être des cellules eucaryotes ou procaryotes. Les cellules peuvent être des cellules primaires ou des cellules immortalisées. Des exemples de cellules eucaryotes utilisables dans l'invention sont des cellules de mammifères, de préférence des cellules humaines, ou végétales. Les cellules mammifères peuvent être par exemple des cellules lymphoïdes, des cellules embryonnaires, des cellules foetales, des cellules épithéliales, des cellules myéloïdes, des cellules tumorales, des cellules souches adultes ou embryonnaires, des cellules infectées par un virus, une bactérie, un champignon ou un parasite. De préférence, les cellules sont des cellules humaines, y compris de donneurs sains. De préférence encore, les cellules sont des cellules tumorales ou des cellules infectées par un virus. Lesdites cellules tumorales peuvent être issues de tumeurs à différents stades d'évolution et/ou de tumeurs présentant différentes sensibilités aux thérapies anticancéreuses. A titre d'exemple non limitatif, les cellules tumorales peuvent être des cellules issues d'un cancer du sein, de la prostate, cérébral (neublastome, astrocytome, oligodendrogliome), du rein, du foie (hépatocarcinome), du pancréas, des glandes surrénales, du colon, un cancer colo-rectal, des ovaires, des testicules, de l'utérus, des poumons, d'un carcinome, d'un sarcome, d'un lymphome, d'un myélome, d'un cancer hématopoiétique, d'une leucémie, d'un mélanome.
Les cellules végétales peuvent être des cellules issues d'une plante monocotylédone ou d'une plante dicotylédone. Par exemple, les cellules végétales peuvent être issues des feuilles, de la tige, de la racine, d'un embryon, d'une pousse, d'un cal.

Les cellules utilisées dans les méthodes de criblage selon l'invention peuvent être des cellules en suspension, des cellules adhérentes en culture à deux dimensions ou en culture à trois dimensions.

### Préparation de la bibliothèque de nanoparticules

L'étape a0) peut être réalisée selon les étapes suivantes (i) préparation et composition d'une formulation sous forme d'une nano-émulsion, comprenant une phase aqueuse continue et au moins une phase dispersée, (ii) encapsulation ou complexation du traceur, (iii) complexation de l'étiquette ADN, (iv) encapsulation ou complexation de la molécule candidate. Les étapes (i) à (iv) peuvent notamment être réalisées selon les méthodes décrites dans l'exemple 1 respectivement aux paragraphes 1.1, 1.5, 1.4 et 1.2.

### Mise en contact des cellules avec une bibliothèque de nanoparticules

La mise en contact des cellules avec la bibliothèque de nanoparticules telle que décrite ci-dessus a pour but de permettre à au moins une nanoparticule de s'intégrer dans les cellules. Elle peut être réalisée par des procédés connus de l'homme du métier. Elle est également basée sur le fait que les cellules sont mises en contact avec la bibliothèque de nanoparticules sans ordonnancement préalable des molécules candidates. Ainsi, les cellules sont par exemple cultivées dans un milieu adapté, puis transfectées collectivement avec la bibliothèque de nanoparticules, par exemple dans un ratio 1/1 (1 nanoparticule/lcellule). Selon un autre exemple, chaque nanoparticule de la bibliothèque est déposée sur un support, par exemple dans un puit d'une plaque de culture, et les cellules sont ajoutées sur ledit support.

De préférence, ladite mise en contact consiste en une étape de transfection des cellules avec la bibliothèque de nanoparticules telle que décrite ci-dessus.

L' étape a) de mise en contact des cellules avec la bibliothèque de nanoparticules peut être réalisée par exemple pendant au moins 30min, 1h, 2h, 3h, 4h, 5h, 6h, 7h, 8h, 9h, 10h, 12h, 18h, 24h, 36h, 48h, 3j, 4j, 5j, 6j, 7j, 8j, 9j, ou 10j, et/ou au plus 10j, 9j, 8j, 7j, 6j, 5j, 4j, 3j, 48h, 36h, 24h, 18h, 12h, 10h, 8h, 7h, 6h, 5h, 4h, 3h, 2h, 1h ou 30min.

L' étape a) de mise en contact des cellules avec la bibliothèque de nanoparticules peut être réalisée par exemple à une température d'au moins 20, 21, 22, 23, 24, 25, 26, 27, 28, 29, 30, 31, 32, 33, 34, 35, 36, 37, 38, 39, ou 40°C, et/ou d'au plus 40, 39, 38, 37, 36, 35, 34, 33, 32, 31, 30, 29, 28, 27, 26, 25, 24, 23, 22, 21, ou 20°C.

Dans un mode de réalisation, lorsque les cellules sont des cellules adhérentes, l'étape a) est suivie d'une étape de trypsination des cellules ou de grattage.

### Sélection des cellules

Dans les méthodes selon l'invention, les cellules sont sélectionnées selon les deux critères suivants : (i) l'intégration du traceur tel que décrit ci-dessus et (ii) la présentation d'un phénotype d'intérêt.

L'identification des cellules ayant intégré le traceur peut être réalisée à l'aide de méthodes connues de l'homme du métier.

Dans un mode de réalisation, lorsque ledit traceur est un traceur magnétique, la sélection des cellules ayant intégré ledit traceur magnétique est réalisée par tri cellulaire magnétique (MACS® pour « Magnetic Activated Cell Sorting » en anglais).

Dans un mode de réalisation, lorsque ledit traceur est un fluorophore, la sélection des cellules ayant intégré ledit fluorophore est réalisée par cytométrie en flux.

L'identification des cellules présentant un phénotype d'intérêt peut être réalisée à l'aide de méthodes connues de l'homme du métier. De préférence, l'identification des cellules présentant un phénotype d'intérêt est réalisée par cytométrie en flux.

Dans un mode de réalisation, l'identification des cellules ayant intégré le traceur est réalisée avant l'identification des cellules présentant un phénotype d'intérêt. Dans un autre mode de réalisation, l'identification des cellules ayant intégré le traceur et l'identification des cellules présentant un phénotype d'intérêt sont réalisées simultanément.

Des exemples non limitatifs de phénotype d'intérêt sont notamment l'apoptose, la prolifération cellulaire, la différenciation, l'expression d'un marqueur d'un cancer, comme par exemple l'expression de l'antigène spécifique de la prostate (PSA pour « Prostate Specific Antigen » en anglais), la résistance ou la sensibilité à un agent thérapeutique, par exemple à un agent de chimiothérapie, la résistance ou la sensibilité à un agent infectieux, de préférence à un virus, la résistance ou la sensibilité à un stress environnemental, comme par exemple la résistance ou la sensibilité à la sécheresse ou à un insecte.

Les phénotypes d'intérêt peuvent être étudiés sans marquage préalable ou avec un marquage à l'aide d'un gène rapporteur fluorescent ou bien encore après marquage à l'aide d'un anticorps fluorescent. L'identification de la prolifération des cellules peut notamment être réalisée par marquage à l'iodure de propidium, à l'EdU, ou à l'Hoechst 33342. L'identification de l'apoptose des cellules peut notamment être réalisée par marquage Annexine V-FITC ou par marquage dit TUNEL (« Terminal deoxynucleotidyl transferase dUTP Nick End Labeling » en anglais) ou par l'analyse de l'activation des caspases.

### Identification de la molécule d'intérêt/candidate

Dans les méthodes selon l'invention, l'identification à l'étape c) des molécules candidates ayant été intégrées à l'étape b) est réalisée en identifiant la séquence de l'étiquette ADN unique. En effet, chaque molécule candidate étant associée à une étiquette ADN unique, l'identification de ladite étiquette ADN unique permet de savoir quelle molécule candidate a été intégrée dans les cellules.

L'identification de l'étiquette ADN est réalisée après (i) extraction de l'ADN des cellules sélectionnées à l'étape b), (ii) amplification de l'ADN extrait à l'étape (i) avec des amorces universelles complémentaires des première et deuxième séquences d'au moins 20 nucléotides desdites étiquettes ADN unique telles que définies ci-dessus, et séquençage de l'ADN amplifié, ou (iib) de séquençage de l'ADN extrait à l'étape (i).

L'étape (i) d'extraction de l'ADN peut être réalisée par des procédés connus de l'homme du métier.

A l'étape (ii), l'amplification peut être réalisée en utilisant des techniques conventionnelles de réaction en chaine par polymérase (PCR pour « Polymerase Chain Reaction » en anglais).

Dans certains modes de réalisation, l'amplification à l'étape (ii) comprend une étape de dénaturation initiale suivie de cycles de dénaturation-hybridation-élongation et d'une étape de d'extension finale.

L'étape de dénaturation initiale peut être réalisée dans des conditions de température allant de 90°C à 105°C, pendant 15 sec à 15 min, de préférence de 92°C à 102°C, de préférence encore de 95°C à 100°C. De préférence, l'étape de dénaturation initiale est réalisée pendant 1 min à 15 min, de préférence encore pendant 2 min à 12 min, de préférence encore pendant 5 min à 10 min.

Chaque cycle de dénaturation-hybridation-élongation inclut une phase de dénaturation dans des conditions de chauffe, suivie d'une phase d'hybridation des amorces réalisée dans des conditions permettant l'hybridation des amorces à l'étiquette ADN unique à amplifier, et une phase d'élongation réalisée dans des conditions permettant à la polymérase de synthétiser un produit d'extension à partir de chaque amorce s'étant hybridée à l'étiquette ADN à amplifier.

La phase de dénaturation peut être réalisée entre 90°C et 105°C, de préférence entre 92°C et 100°C, de préférence encore entre 94°C et 98°C, pendant 10 sec à 4 min, de préférence pendant 10 sec à 2 min, de préférence encore pendant 15 sec à 1 min.

La phase d'hybridation, c'est-à-dire la phase hybridation des amorces, peut être réalisée entre 35°C et 70°C, de préférence entre 40°C à 65°C, de préférence encore entre 45°C à 60°C, pendant 10 sec à 2 min, de préférence pendant 20 sec à 1,5 min, de préférence encore pendant 25 sec à 45 sec.

La phase d'élongation peut être réalisée entre 40°C et 80°C, de préférence entre 50°C et 75°C, de préférence encore entre 60°C et 72°C, pendant 10 sec à 5 min, de préférence pendant 20 sec à 3 min, de préférence encore pendant 25 sec à 1 min.

Les étapes de dénaturation-hybridation-élongation peuvent être répétées pendant 30 à 60 cycles, de préférence pendant 35 à 45 cycles.

L'étape d'extension finale peut être réalisée entre 40°C et 80°C, de préférence entre 50°C et 75°C, de préférence encore entre 60°C et 72°C, pendant 1 min à 10 min, de préférence pendant 3 min à 8 min, de préférence encore pendant 4 min à 6 min.

Le séquençage aux étapes (ii) et (iib) peut être réalisée en utilisant des techniques conventionnelles de séquençage.

De préférence, l'étape (iib) de séquençage est réalisée sur des séquenceurs de troisième génération qui permettent le séquençage d'une seule molécule d'ADN, e.g. en utilisant la méthode Pacific Bioscience®, la méthode ion torrent™, la méthode Oxford nanopore®, ou la méthode dite « optipore » (Noblegen BioSciences). Le séquençage aux étapes (ii) et (iib) permet donc d'identifier la séquence unique d'au moins 10 nucléotides de chaque étiquette ADN.

### Crible individuel des molécules candidates

Dans les méthodes de criblages selon l'invention, il est possible que les cellules intègrent plusieurs nanoparticules, ceci conduisant à identifier à l'étape c) plusieurs molécules candidates comme étant responsables du phénotype d'intérêt. Ainsi afin de vérifier si toutes les molécules candidates identifiées sont réellement responsables du phénotype d'intérêt, les méthodes de criblage selon l'invention peuvent donc, après l'étape c), en outre, comprendre une étape de criblage individuel des molécules candidates identifiées à l'étape c).

Cette étape de criblage individuel est effectuée en réalisant un second cycle d'étapes a) à c), étant entendu que la mise en contact des cellules à l'étape a) est réalisée avec une nanoparticule comprenant un traceur, un fluorophore et la molécule candidate identifiée à l'étape c).

### Formulation dans une composition pharmaceutique

La méthode de criblage d'une molécule d'intérêt selon l'invention peut en outre comprendre après l'étape c), ou après l'étape de criblage individuel lorsque celle-ci est présente, une ou plusieurs étapes supplémentaires consistant à formuler ladite molécule d'intérêt dans une composition pharmaceutique avec un véhicule pharmaceutiquement acceptable.

Par « véhicule pharmaceutiquement acceptable », on entend un véhicule adapté pour une utilisation en contact avec des cellules humaines ou animales, sans induire de toxicité, irritation, ou réponse allergique indue. Des exemples non limitatifs de véhicules pharmaceutiquement acceptables incluent notamment une solution physiologique ou des liposomes, des microparticules, des microcapsules ou des systèmes de transport basés sur des lipides. Les liposomes, microparticules, microcapsules et systèmes de transport basés sur des lipides sont des véhicules pharmaceutiquement acceptables particulièrement adaptés pour être formulés avec des acides nucléiques.

### Maladie / Trait phénotypique

Dans certains modes de réalisation, la maladie est un cancer, une infection ou une maladie cardio-vasculaire.

De préférence, le cancer est choisi parmi un cancer du sein, de la prostate, cérébral (neublastome, astrocytome, oligodendrogliome), du rein, du foie (hépatocarcinome), du pancréas, des glandes surrénales, du colon, un cancer colo-rectal, des ovaires, des testicules, de l'utérus, des poumons, un carcinome, un sarcome, un lymphome, un myélome, un cancer hématopoïétique, une leucémie ou un mélanome. De préférence la maladie est un cancer de la prostate.

De préférence, l'infection est choisie parmi une infection bactérienne, parasitaire ou virale. De préférence, l'infection est une infection virale.

La maladie cardio-vasculaire est choisie parmi l'hypertension, le syndrome coronaire aigu, l'athérosclérose, la calcification vasculaire, ou l'accident vasculaire cérébral.

Le terme « trait phénotypique » se réfère à l'apparence ou à une autre caractéristique d'un organisme résultant de l'interaction du génome dudit organisme avec l'environnement. Dans certains modes de réalisation, le trait phénotypique est choisi parmi l'apoptose, la prolifération cellulaire, la résistance ou la sensibilité à un agent thérapeutique, par exemple à un agent de chimiothérapie, la résistance ou la sensibilité à un agent infectieux, de préférence à un virus, la résistance ou la sensibilité à un stress environnemental, comme par exemple la résistance ou la sensibilité à la sécheresse, au froid, au gel, aux hautes températures, la résistance ou la sensibilité à un insecte, la résistance ou la sensibilité à un herbicide, le rendement, la taille.

### Identification du biomarqueur de la maladie ou du trait phénotypique

Dans la méthode de criblage d'un biomarqueur d'une maladie ou d'un trait phénotypique selon l'invention, l'identification à l'étape d) du biomarqueur de la maladie ou du trait phénotypique est réalisée en identifiant la cible de la molécule candidate identifiée à l'étape c).

Par « cible », on entend un gène, un ARN ou une protéine dont l'expression est modulée par une molécule candidate.

Dans la mesure où la modulation de ladite cible est responsable du phénotype d'intérêt, et donc de la maladie ou du trait phénotypique, ladite cible est donc identifiée comme étant un biomarqueur de ladite maladie ou dudit trait phénoypique.

### Définitions

Dans le cadre de la présente invention, on entend par le terme « nanoparticule » une particule de moins de 1 micron de diamètre. Dans certains modes de réalisation, ladite nanoparticule a un diamètre moyen d'au moins 10, 20, 30, 40, 50, 60, 70, 80, 90, 100, 150, 200, 250, 300 nm ou d'au plus 300, 250, 200, 150, 100, 90, 80, 70, 60, 50, 40, 30, 20, 10 nm. Dans certains modes de réalisation, ladite nanoparticule a un diamètre moyen compris entre 10 nm et 500 nm, de préférence entre 20 nm et 200 nm, de préférence encore entre 50 et 150 nm.

Dans le cadre de la présente demande, on entend par le terme « nanoémulsion » une composition présentant au moins deux phases, généralement une phase huileuse et une phase aqueuse, dans laquelle la taille moyenne de la phase dispersée est inférieure à 1 micron, de préférence de 10 à 500 nm et en particulier de 20 à 200 nm, et tout préférentiellement de 50 à 200 nm (voir articles C. Solans, P. Izquierdo, J. Nolla, N. Azemar et M. J. Garcia-Celma, Curr Opin Colloid In, 2005, 10, 102-110).

Au sens de la présente demande, l'expression « phase dispersée » signifie les gouttelettes comprenant l'éventuelle huile/ le(s) tensioactif(s) cationique(s) / le lipide solubilisant/ le lipide amphiphile/ le co-tensioactif/ l'éventuel tensioactif de formule (I)/ l'éventuel lipide fusogène / la molécule candidate / le fluorophore / l'étiquette ADN unique. La phase dispersée est généralement exempte de phase aqueuse.

Le terme « gouttelette » englobe à la fois les gouttelettes d'huile liquide proprement dites ainsi que les particules solides issues d'émulsions de type huile-dans-eau dans lesquelles la phase dispersée est solide. Dans ce dernier cas, on parle souvent aussi d'émulsion solide.

Le terme « lipide » désigne dans le cadre de cet exposé l'ensemble des corps gras ou des substances contenant des acides gras présents dans les graisses d'origine animales et dans les huiles végétales. Ce sont des molécules hydrophobes ou amphiphiles principalement constituées de carbone, d'hydrogène et d'oxygène et ayant une densité inférieure à celle de l'eau. Les lipides peuvent être à l'état solide à température ambiante (25°C), comme dans les cires, ou liquide, comme dans les huiles.

Le terme « phospholipide » vise des lipides possédant un groupe phosphate, notamment les phosphoglycérides. Le plus souvent, les phospholipides comportent une extrémité hydrophile formée par le groupe phosphate éventuellement substitué et deux extrémités hydrophobes formées par des chaînes d'acides gras. Parmi les phospholipides, on citera en particulier la phosphatidylcholine, la phosphatidyl éthanolamine, la phophatidyl inositol, la phosphatidyl sérine et la sphingomyéline.

Le terme « lécithine » désigne la phosphatidylcholine, c'est-à-dire un lipide formé à partir d'une choline, d'un phosphate, d'un glycérol et de deux acides gras. Il couvre de manière plus large les phospholipides extraits du vivant, d'origine végétale ou animale, dans la mesure où ils sont majoritairement constitués de phosphatidylcholine. Ces lécithines constituent généralement des mélanges de lécithines portant différents acides gras.

On entend par le terme « acide gras » désigner des acides carboxyliques aliphatiques présentant une chaîne carbonée d'au moins 4 atomes de carbone. Les acides gras naturels possèdent une chaîne carbonée de 4 à 28 atomes de carbone (généralement un nombre pair). On parle d'acide gras à longue chaîne pour une longueur de 14 à 22 carbones et à très longue chaîne s'il y a plus de 22 carbones.

On entend par le terme « tensioactif » des composés à structure amphiphile qui leur confère une affinité particulière pour les interfaces de type huile/eau et eau/huile ce qui leur donne la capacité d'abaisser l'énergie libre de ces interfaces et de stabiliser des systèmes dispersés.

On entend par le terme « co-tensioactif » un tensioactif agissant en plus d'un tensioactif pour abaisser davantage l'énergie de l'interface.

On entend par le terme « chaîne hydrocarbonée » désigner une chaîne composée d'atomes de carbone et d'hydrogène, saturée ou insaturée (double ou triple liaison). Les chaînes hydrocarbonées préférées sont les alkyle ou alcényle.

On entend par le terme « alkylène » désigner un groupe divalent aliphatique hydrocarboné, saturé, linéaire ou ramifié, de préférence linéaire.

L'invention sera décrite plus en détail au moyen des figures en annexe et des exemples qui suivent.

### FIGURES

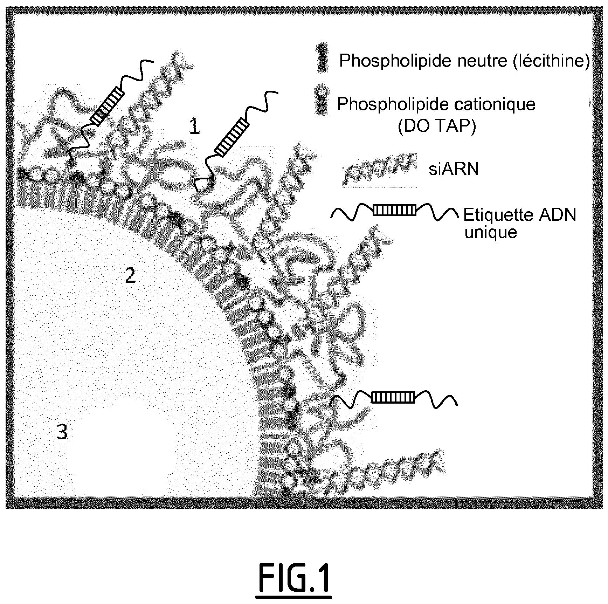

La figure 1 représente un schéma illustrant la structure coeur/couronne d'une gouttelette d'une formulation sous forme d'une nano-émulsion, comprenant une phase aqueuse continue et au moins une phase dispersée, telle que définie dans l'invention, et comprenant une séquence nucléotidique susceptible de moduler des mécanismes endogènes d'ARN interférence, un traceur et une étiquette ADN unique. 1 représente la phase aqueuse continue, 2 la couronne (en partie) de la gouttelette et 3 le coeur (en partie) de la gouttelette. Dans la couronne 2, sont illustrés : le lipide amphiphile (par exemple phospholipide neutre tel que la lécithine), le tensioactif cationique (phospholipide cationique DOTAP par exemple), le co-tensioactif dont la chaîne poly(oxyde d'éthylène) se repliant est représentée dans la phase aqueuse, la séquence nucléotidique double brin susceptible de moduler des mécanismes endogènes d'ARN interférence (siARN par exemple) et l'étiquette ADN unique.
La figure 2 représente un gel d'électrophorèse révélé à l'UV réalisé après complexation de siARN avec la formulation B10 avec des concentrations précisées au tableau 8. L'échelle et les siARN (témoin) sont à gauche.
La figure 3 donne les résultats de quantité de SiARN (ng) obtenus par traitement des données de l'électrophorèse sur le logiciel ImageJ du gel d'électrophorèse révélé à l'UV de la figure 2.
La figure 4 représente l'intensité obtenue sur un appareil ZetaSizer Malvern en fonction du diamètre hydrodynamique en nm pour le siARN libre (comparaison) et pour trois formulations selon l'invention obtenues par complexation avec un rapport N/P : quantité de charges positives dues au tensioactif cationique dans la formulation « prémix » (dues à l'azote chargé, d'où le N de N/P), sur la quantité de charges négatives apportée par les siARN (dues au phosphore du siARN, d'où le P de N/P) de 4/1, de 8/1 ou de 16/1.
La figure 5 représente un gel d'électrophorèse révélé à l'UV avec, de gauche à droite :
   - l'échelle,
   - le siARN libre (témoin)
   puis des migrations obtenues par complexation de siARN :
   - avec la formulation B1 (exemple comparatif) (pas de complexation, les siARN sont libres),
   - avec la formulation B6 avec un rapport quantité de charges positives dues au tensioactif cationique dans la formulation sur la quantité de charge négative apportée par les siARN de 8/1 (complexation quantitative),
   - avec la formulation B6 avec un rapport quantité de charges positives dues au tensioactif cationique dans la formulation sur la quantité de charge négative apportée par les siARN de 8/1 (complexation quantitative),
   - avec la formulation B10 avec un rapport quantité de charges positives dues au tensioactif cationique dans la formulation sur la quantité de charge négative apportée par les siARN de 8/1 (complexation quantitative),
   - avec de la lipofectamine (exemple comparatif) (complexation incomplète).

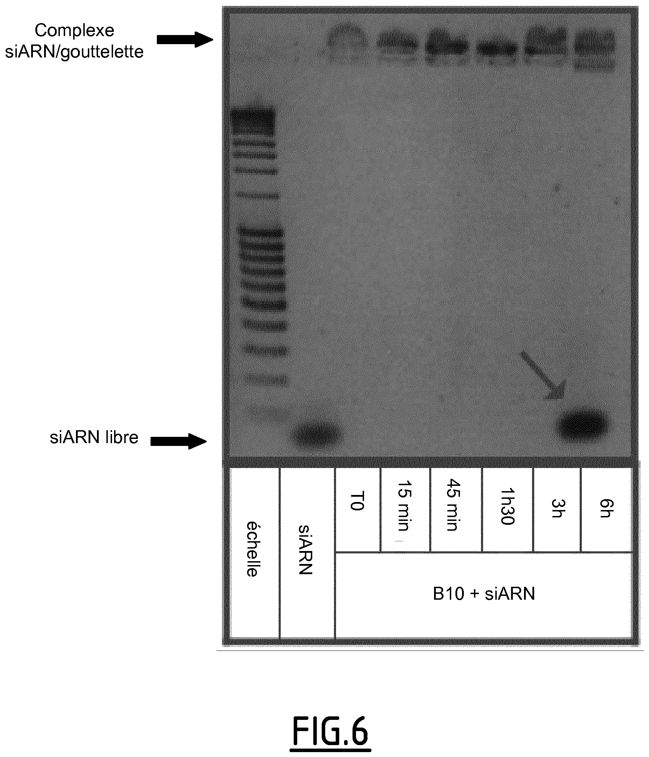

La figure 6 représente un gel d'électrophorèse révélé à l'UV d'un complexe siARN/formulation B10 juste après complexation (T0) puis 15 min, 45 min, 1h30, 3h et 6h après complexation. L'échelle et les siARN (témoin) sont à gauche.

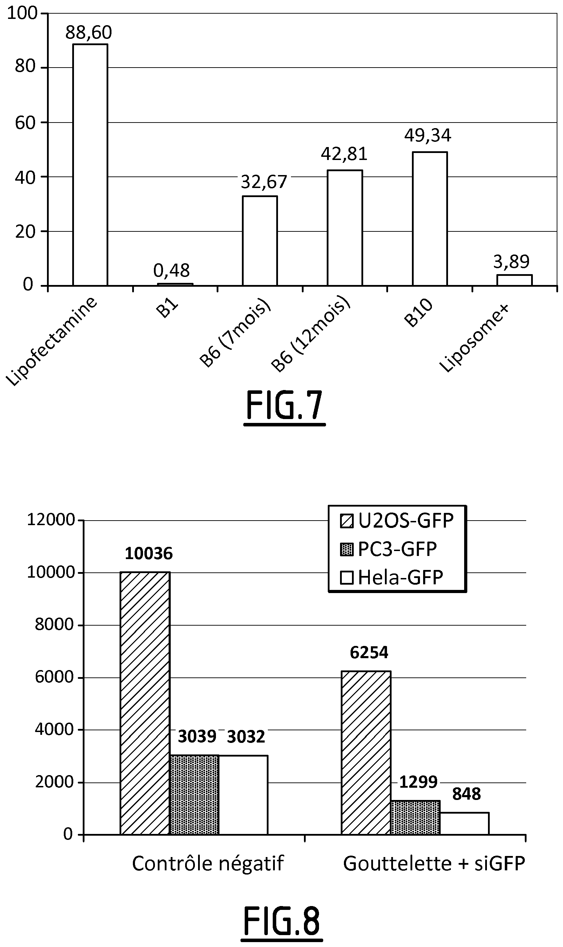

La figure 7 représente la diminution de l'intensité de fluorescence FITC en % en transfectant les lignées cellulaires avec :
   - de la Lipofectamine RNAimax (agent commercial),
   - le complexe siARN/formulation B1,
   - avec le complexe siARN/formulation B6, la formulation ayant été conservée 7 mois à température ambiante préalablement à la complexation,
   - avec le complexe siARN/formulation B6, la formulation ayant été conservée 12 mois à température ambiante préalablement à la complexation,
   - avec le complexe siARN/formulation B10, la formulation ayant été conservée 12 mois à température ambiante préalablement à la complexation,
   - avec un complexe siARN / formulation de liposomes cationiques comprenant du DOTAP (58% wt), du DOPE (18% wt), du cholestérol (2% wt) et du DSPE-PEG3000 (22% wt)) (comparatif).
La figure 8 représente l'intensité de fluorescence (FITC) pour 3 lignées cellulaires surexprimant expérimentalement la protéine fluorescente GFP (pour green fluorescent protein) : U2OS, PC3 et Hela pour le complexe Si/ARN formulation B10, le siARN appelé siGFP étant ciblé contre l'ARNm codant pour la protéine GFP, et pour le contrôle négatif (cellules incubées avec une formulation B10 complexée avec un siRNA contrôle négatif, c'est-à-dire « inerte » sans effet sur le transcriptome de la cellule appelé siAllStar).

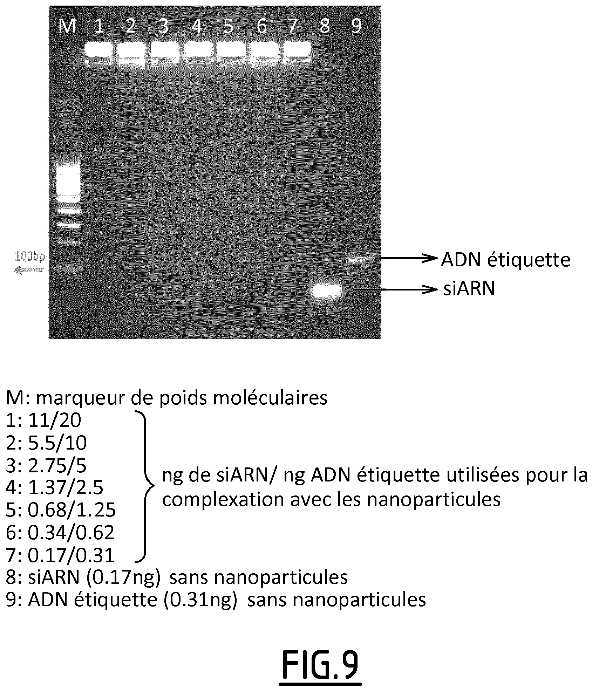

La figure 9 représente un gel d'électrophorèse révélant les complexes nanoparticules A3/étiquette ADN/siARN. Les acides nucléiques sont révélés à l'aide du réactif GelRed Nucleic Acid Gel Stain (Interchim, Ref BY1740)

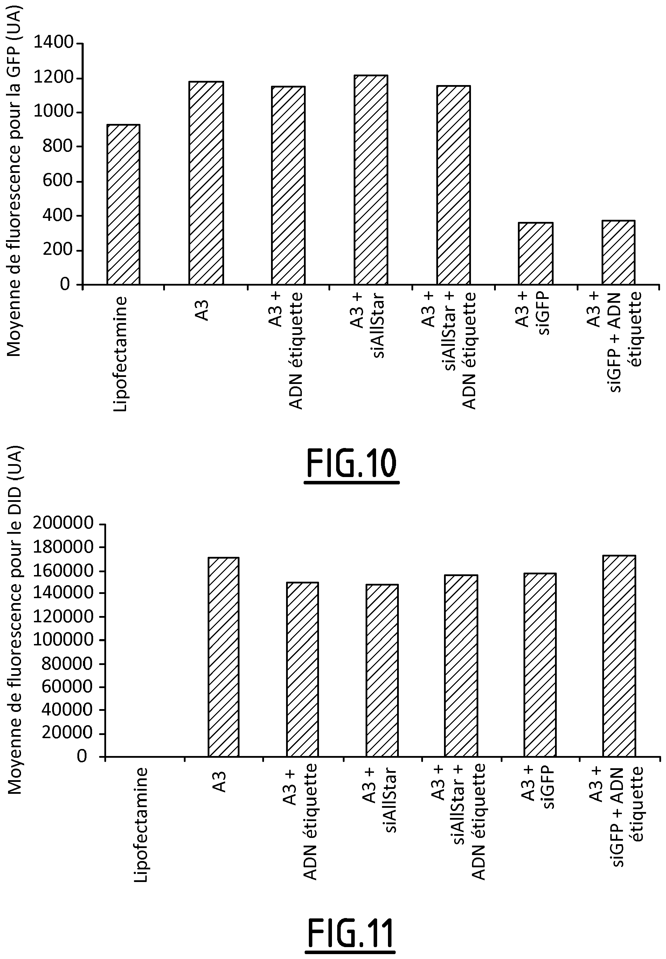

La figure 10 représente la quantification des moyennes de fluorescences pour la GFP après analyse FACS des cellules HeLa et incubées pendant 72h avec les complexes nanoparticules A3/étiquette ADN/siGFP.
La figure 11 représente la quantification des moyennes de fluorescences pour le fluorophore DID encapsulé après analyse FACS des cellules HeLa et incubées pendant 72h avec les complexes nanoparticules A3/étiquette ADN/siGFP.

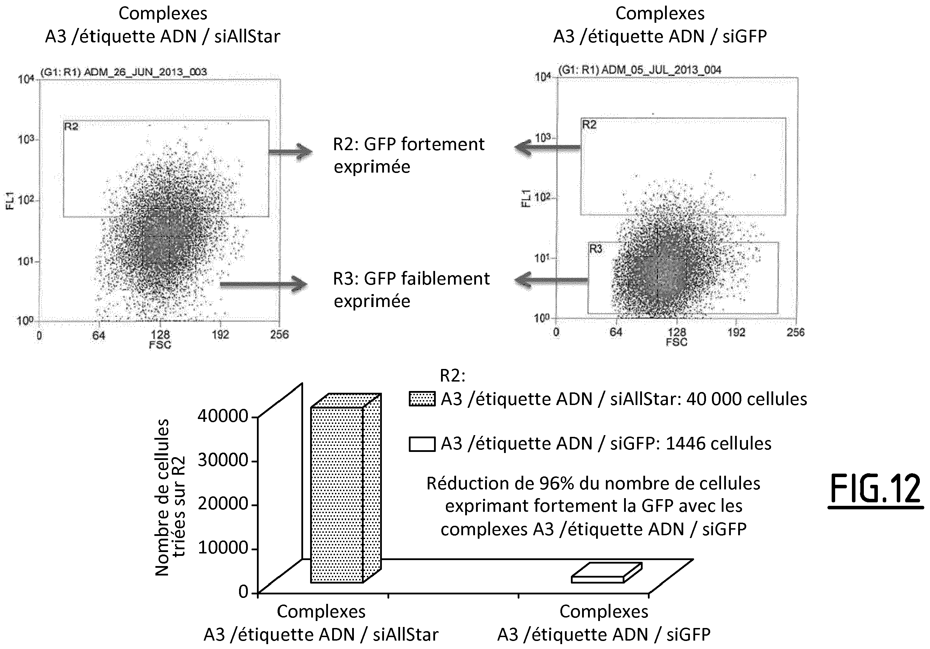

La figure 12 représente l'analyse FACS du signal FITC correspondant à l'expression de la GFP par les cellules HeLa. Le tri de ces cellules a été fait selon leur critère d'expression de la GFP et permet d'identifier deux populations : une population qui exprime fortement la GFP et une population qui exprime faiblement la GFP.

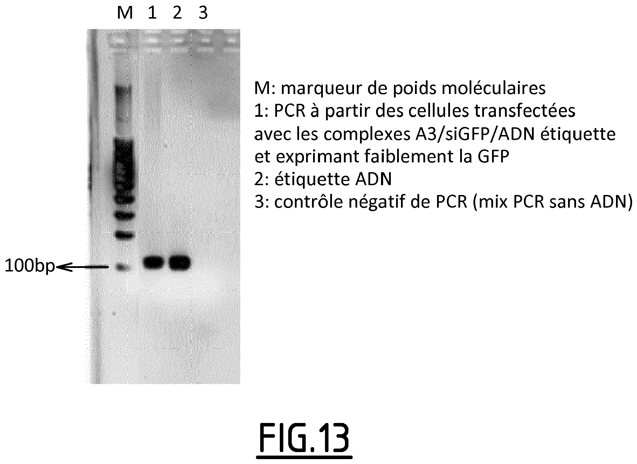

La figure 13 représente un gel d'électrophorèse des ADN étiquettes après extraction et amplification par PCR sur des cellules HeLa préalablement triées.

### EXEMPLES

### Exemple 1 : Conception et préparation de nanoparticules fluorescentes contenant une étiquette ADN et un siARN.

### 1.1. Préparation et composition de formulations sous forme d'une nano-émulsion, comprenant une phase aqueuse continue et au moins une phase dispersée

La phase aqueuse utilisée est une solution tampon PBS 1X.

Les fournisseurs des composés sont les suivants :
Lipoid S75-3 : Lipoid
Lipoid S75 : Lipoid
Lipoid S100-3 : Lipoid
DOTAP : Avanti Polar
DOPE : Avanti Polar
MyrjS40 : Croda
Suppocire NB : Gattefossé
Huile de soja : Croda

Le diamètre hydrodynamique des gouttelettes des formulations ainsi que leur potentiel zeta ont été mesurés par diffusion quasi-élastique de la lumière par un appareil de type ZetaSizer, Malvern. Le diamètre hydrodynamique des gouttelettes a été mesuré dans une solution de PBS 0,1X, le potentiel zeta dans une solution aqueuse de NaCl 0,15 mM.

Seize formulations différentes ont été réalisées, dont les compositions sont indiquées dans les tableaux 1 à 5.

**Tableau 1**

| | | **A1 (comp.)** | **A2** | **A3** |
|---|---|---|---|---|
| **COURONNE** | **Lipide amphiphile : Lipoid** | S75-3 | S75-3 | S75-3 |
| | ***% wt Lipoid* / *gouttelette*** | 35,29 | 8,83 | 3,53 |
| | *% wt Lipoid*/*couronne sans PEG* | 100 | 25 | 10 |
| | *% wt Lipoid*/*couronne* | 46,15 | 11,54 | 4,62 |
| | *% mol. Lipoid*/*couronne* | 70,02 | 16,86 | 6,74 |
| | **Tensioactif cationique DOTAP** | X | DOTAP | DOTAP |
| | ***% wt DOTAP* / *gouttelette*** | 0 | 26,47 | 26,47 |
| | *% wt DOTAP*/*couronne sans PEG* | 0 | 75 | 75 |
| | *% wt DOTAP* / *couronne* | 0 | 34,61 | 34,61 |
| | ***% mol. DOTAP* / *couronne*** | **0** | **54,28** | **54,24** |
| | **Co-tensioactif** | Myrj S40 | Myrj S40 | Myrj S40 |
| | ***% wt co-tensioactif*/*gouttelette*** | 41,17 | 41,17 | 41,17 |
| | *% wt (co-tensioactif)* / *couronne* | 53,85 | 53,85 | 53,85 |
| | ***% mol. (co-tensioactif)* / *couronne*** | **29,98** | **28,86** | **28,84** |
| | **Lipide fusogène DOPE** | X | X | DOPE |
| | ***% wt DOPE* / *gouttelette*** | 0 | 0 | 5,29 |
| | *% wt DOPE*/*couronne sans PEG* | 0 | 0 | 15 |
| | *% wt DOPE* / *couronne* | 0 | 0 | 6,92 |
| | *% mol. DOPE* / *couronne* | 0 | 0 | 10,18 |
| **COEUR** | **Lipide solubilisant** | Suppocire NB | Suppocire NB | Suppocire NB |
| | *% wt lipide solubilisant*/*coeur* | 75 | 75 | 75 |
| | ***% wt lipide solubilisant* / *gouttelette*** | 17,65 | 17,65 | 17,65 |
| | **Huile** | Huile de soja | Huile de soja | Huile de soja |
| | *% wt huile* / *coeur* | 25 | 25 | 25 |
| | ***% wt huile* / *gouttelette*** | 5,88 | 5,88 | 5,88 |
| | **Formulation (nombre de répétition)** | 3 | 4 | 3 |
| **RESULTAT** | **Diamètre hydrodynamique (nm) - average** | 124,9 | 49,1 | 44,48 |
| | **ZP (mV)_dans NaCl 0,15 mM** | -26 | 25,38 | 30,87 |
| | **Stabilité** | ok | ok | ok |
| | **Complexation (%)** | 0 | 100 | 100 |
| | **Efficacité d'extinction (%)** | 0 | 72,88 | 75,06 |

**Tableau 2**

| | | **B1 (comp.)** | **B2** | **B3** |
|---|---|---|---|---|
| **COURONNE** | **Lipide amphiphile : Lipoid** | S75-3 | S75 | S100-3 |
| | ***% wt Lipoid* / *gouttelette*** | 8,75 | 4,25 | 4,25 |
| | *% wt Lipoid* /*couronne sans PEG* | 100 | 50 | 50 |
| | *% wt Lipoid* / *couronne* | 15,98 | 7,99 | 7,99 |
| | *% mol. Lipoid* / *couronne* | 34,14 | 17,89 | 17,89 |
| | **Tensioactif cationique DOTAP** | X | DOTAP | DOTAP |
| | ***% wt DOTAP* / *gouttelette*** | 0 | 4,25 | 4,25 |
| | *% wt DOTAP* /*couronne sans PEG* | 0 | 50 | 50 |
| | *% wt DOTAP* / *couronne* | 0 | 7,99 | 7,99 |
| | ***% mol. DOTAP* / *couronne*** | 0 | **17,85** | **17,85** |
| | **Co-tensioactif** | Myrj S40 | Myrj S40 | Myrj S40 |
| | ***% wt co-tensioactif*/ *gouttelette*** | 46 | 46 | 46 |
| | *% wt (co-tensioactif)* / *couronne* | 84,02 | 84,02 | 84,02 |
| | ***% mol. (co-tensioactif)* / *couronne*** | **65,86** | **64,27** | **64,27** |
| | **Lipide fusogène DOPE** | X | X | X |
| | ***% wt DOPE*/*gouttelette*** | 0 | 0 | 0 |
| | *% wt DOPE* /*couronne sans PEG* | 0 | 0 | 0 |
| | *% wt DOPE* / *couronne* | 0 | 0 | 0 |
| | *% mol. DOPE* / *couronne* | 0 | 0 | 0 |
| **COEUR** | **Lipide solubilisant** | Suppocire NB | Suppocire NB | Suppocire NB |
| | *% wt lipide solubilisant*/*coeur* | 75 | 75 | 75 |
| | ***% wt lipide solubilisant* / *gouttelette*** | 33,94 | 33,94 | 33,94 |
| | **Huile** | Huile de soja | Huile de soja | Huile de soja |
| | *% wt huile* / *coeur* | 25 | 25 | 25 |
| | ***% wt huile* / *gouttelette*** | 11,31 | 11,31 | 11,31 |
| | **Formulation (nombre de répétition)** | 6 | 2 | 2 |
| **RESULTAT** | **Diamètre hydrodynamique (nm) - average** | 59,51 | 42,11 | 61,43 |
| | **ZP (mV)_dans NaCl 0,15 mM** | -21,8 | 21,4 | 6,72 |
| | **Stabilité** | ok | ok | ok |
| | **Complexation (%)** | 0 | ND | ND |
| | **Efficacité d'extinction (%)** | 0 | 0 | 0 |

**Tableau 3**

| | | **B4 (comp.)** | **B5** | **B6** |
|---|---|---|---|---|
| **COURONNE** | **Lipide amphiphile : Lipoid** | S75-3 | S75-3 | S75-3 |
| | ***% wt Lipoid* / *gouttelette*** | 6,58 | 2,19 | 2,84 |
| | *% wt Lipoid* /*couronne sans PEG* | 100 | 25 | 25 |
| | *% wt Lipoid* / *couronne* | 14,29 | 4 | 6,9 |
| | *% mol. Lipoid* / *couronne* | 31,24 | 8,39 | 12,39 |
| | **Tensioactif cationique DOTAP** | X | DOTAP | DOTAP |
| | ***% wt DOTAP* / *gouttelette*** | 0 | 6,56 | 8,52 |
| | *% wt DOTAP* /*couronne sans PEG* | 0 | 75 | 75 |
| | *% wt DOTAP* / *couronne* | 0 | 11,99 | 20,67 |
| | ***% mol. DOTAP* / *couronne*** | **0** | **26,99** | **39,87** |
| | **Co-tensioactif** | Myrj S40 | Myrj S40 | Myrj S40 |
| | ***% wt co-tensioactif*/*gouttelette*** | 39,48 | 46 | 29,87 |
| | *% wt (co-tensioactif)* / *couronne* | 85,71 | 84,02 | 72,43 |
| | ***% mol. (co-tensioactif)* / *couronne*** | **68,76** | **64,63** | **47,74** |
| | **Lipide fusogène DOPE** | X | X | X |
| | *%* ***wt DOPE* / *gouttelette*** | 0 | 0 | 0 |
| | *% wt DOPE*/*couronne sans PEG* | 0 | 0 | 0 |
| | *% wt DOPE* / *couronne* | 0 | 0 | 0 |
| | *% mol. DOPE* / *couronne* | 0 | 0 | 0 |
| **COEUR** | **Lipide solubilisant** | Suppocire NB | Suppocire NB | Suppocire NB |
| | *% wt lipide solubilisant*/*coeur* | 75 | 75 | 75 |
| | ***% wt lipide solubilisant* / *gouttelette*** | 40,46 | 33,94 | 44,07 |
| | **Huile** | Huile de soja | Huile de soja | Huile de soja |
| | *% wt huile* / *coeur* | 25 | 25 | 25 |
| | **% *wt huile* / *gouttelette*** | 13,49 | 11,31 | 14,69 |
| | **Formulation (nombre de répétition)** | 3 | 4 | 6 |
| **RESULTAT** | **Diamètre hydrodynamique (nm) - average** | 84,88 | 56,68 | 86,77 |
| | **ZP (mV)_dans NaCl 0,15 mM** | -18,89 | 26,51 | 36,38 |
| | **Stabilité** | ok | ok | ok |
| | **Complexation (%)** | 0 | 100 | 100 |
| | **Efficacité d'extinction (%)** | 0 | 0 | 42,81 |

**Tableau 4**

| | | **B9 (Comp.)** | **B10** |
|---|---|---|---|
| **COURONNE** | **Lipide amphiphile : Lipoid** | S75-3 | S75-3 |
| | ***% wt Lipoid* / *gouttelette*** | 0 | 1,71 |
| | *% wt Lipoid* /*couronne sans PEG* | 0 | 15 |
| | *% wt Lipoid* / *couronne* | 0 | 4,14 |
| | *% mol. Lipoid* / *couronne* | 0 | 7,43 |
| | **Tensioactif cationique DOTAP** | DOTAP | DOTAP |
| | ***% wt DOTAP* / *gouttelette*** | 8,25 | 8,25 |
| | *% wt DOTAP* /*couronne sans PEG* | 75 | 75 |
| | *% wt DOTAP* / *couronne* | 20,67 | 20,67 |
| | ***% mol. DOTAP* / *couronne*** | **39,87** | **39,87** |
| | **Co-tensioactif** | Myrj S40 | Myrj S40 |
| | ***% wt co-tensioactif*/ *gouttelette*** | 29,87 | 29,87 |
| | *% wt (co-tensioactif)* / *couronne* | 72,43 | 72,43 |
| | ***% mol. (co-tensioactif)* / *couronne*** | **47,74** | **47,74** |
| | **Lipide fusogène DOPE** | DOPE | DOPE |
| | ***% wt DOPE* / *gouttelette*** | 2,84 | 1,71 |
| | *% wt DOPE* /*couronne sans PEG* | 25 | 10 |
| | *% wt DOPE* / *couronne* | 6,9 | 2,76 |
| | *% mol. DOPE* / *couronne* | 10,18 | 4,99 |
| **COEUR** | **Lipide solubilisant** | Suppocire NB | Suppocire NB |
| | *% wt lipide solubilisant*/*coeur* | 75 | 75 |
| | ***% wt lipide solubilisant* / *gouttelette*** | 44,07 | 44,07 |
| | **Huile** | Huile de soja | Huile de soja |
| | *% wt huile* / *coeur* | 25 | 25 |
| | ***% wt huile* / *gouttelette*** | 14,69 | 14,69 |
| | **Formulation (nombre de répétition)** | 1 mauvaise formulation | 5 |
| **RESULTAT** | **Diamètre hydrodynamique (nm) - average** | ND | 88,64 |
| | **ZP (mV)_dans NaCl 0,15 mM** | ND | 36,9 |
| | **Stabilité** | ND | Ok |
| | **Complexation (%)** | ND | 100 |
| | **Efficacité d'extinction (%)** | ND | 49,34 |

**Tableau 5**

| | | **C1(comp.)** | **C2** | **C3** |
|---|---|---|---|---|
| **COURONNE** | **Lipide amphiphile : Lipoid** | S75-3 | S75-3 | S75-3 |
| | ***% wt Lipoid* / *gouttelette*** | 28,44 | 7,11 | 4,27 |
| | *% wt Lipoid* /*couronne sans PEG* | 100 | 25 | 15 |
| | *% wt Lipoid* / *couronne* | 70,24 | 17,56 | 10,54 |
| | *% mol. Lipoid* / *couronne* | 86,55 | 20,65 | 12,38 |
| | **Tensioactif cationique DOTAP** | X | DOTAP | DOTAP |
| | ***% wt DOTAP* / *gouttelette*** | 0 | 21,33 | 21,33 |
| | *% wt DOTAP*/*couronne sans PEG* | 0 | 75 | 75 |
| | *% wt DOTAP* / *couronne* | 0 | 52,68 | 52,68 |
| | ***% mol. DOTAP* / *couronne*** | **0** | **66,51** | **66,47** |
| | **Co-tensioactif** | Myrj S40 | Myrj S40 | Myrj S40 |
| | ***% wt co-tensioactif*/ *gouttelette*** | 12,05 | 12,05 | 12,05 |
| | *% wt (co-tensioactif)* / *couronne* | 29,76 | 29,76 | 29,76 |
| | ***% mol. (co-tensioactif)* / *couronne*** | **13,45** | **12,84** | **12,83** |
| | **Lipide fusogène DOPE** | X | X | DOPE |
| | ***% wt DOPE*/ *gouttelette*** | 0 | 0 | 2,84 |
| | *% wt DOPE*/*couronne sans PEG* | 0 | 0 | 10 |
| | *% wt DOPE* / *couronne* | 0 | 0 | 7,02 |
| | *% mol. DOPE* / *couronne* | 0 | 0 | 8,32 |
| **COEUR** | **Lipide solubilisant** | Suppocire NB | Suppocire NB | Suppocire NB |
| | *% wt lipide solubilisant*/*coeur* | 75 | 75 | 75 |
| | ***% wt lipide solubilisant* / *gouttelette*** | 44,63 | 44,63 | 44,63 |
| | **Huile** | Huile de soja | Huile de soja | Huile de soja |
| | *% wt huile* / *coeur* | 25 | 25 | 25 |
| | ***% wt huile* / *gouttelette*** | 14,88 | 14,88 | 14,88 |
| | **Formulation (nombre de répétition)** | 4 | 4 | 3 |
| **RESULTAT** | **Diamètre hydrodynamique (nm) - average** | 153,03 | 162,2 | 168,9 |
| | **ZP (mV)_dans NaCl 0,15 mM** | -37,71 | 53,7 | 51,83 |
| | **Stabilité** | ok | ok | ok |
| | **Complexation (%)** | 0 | 100 | 100 |
| | **Efficacité d'extinction (%)** | 0 | 80,51 | 81,72 |

Dans les tableaux 1 à 5 :
% wt correspond à un pourcentage massique.
% mol. correspond à un pourcentage molaire.
ND (non déterminé) signifie que l'expérience n'a pas été réalisée

Les pourcentages « /gouttelette » représentent des pourcentages par rapport à l'ensemble (Lipoid/DOTAP/Myrj S40/ éventuel DOPE/Suppocire NB/Huile de soja).
Les pourcentages « /couronne » représentent des pourcentages par rapport à l'ensemble (Lipoid/DOTAP/Myrj S40/éventuel DOPE).
Les pourcentages « /couronne sans PEG » représentent des pourcentages par rapport à l'ensemble (Lipoid/DOTAP/éventuel DOPE).
Les pourcentages « /coeur » représentent des pourcentages par rapport à l'ensemble (Suppocire NB/Huile de soja).
Le Lipoid S75-3 comprend 65-75% de phosphatidylcholine. Les chaines aliphatiques des phospholipides sont majoritairement saturées (composition moyenne : 12-16 % de C16:0, 80-85% de C18:0, < 5 % de C18:1, < 2 % de C18:2).
Le Lipoid S75 comprend 65-75% de phosphatidylcholine. Les chaines aliphatiques des phospholipides sont majoritairement insaturées (composition moyenne: 17-20 % de C16:0, 2-5 % de C18:0, 8-12 % de C18:1, 58-65 % de C18:2, 4-6 % de C18:3).
Le Lipoid S100-3 comprend >94 % de phosphatidylcholine. Les chaines aliphatiques des phospholipides sont majoritairement saturées (composition moyenne : 12-16 % de C16:0, 85-88% de C18:0, < 2 % de C18:1, < 1 % de C18:2).

Les formulations A1, B1, B4 et C1 sont des exemples comparatifs, car elles ne comprennent pas de co-tensioactif cationique.
Leurs potentiels zêta sont négatifs.
La complexation de siARN n'a pas eu lieu en surface des gouttelettes, conformément à ce qui était attendu.

La formulation B9 est un exemple comparatif, car elle ne comprend pas de lipide amphiphile. L'émulsion n'a pas pu être préparée.

Le procédé de préparation suivant a été suivi :

### (i) Préparation de la phase huileuse :

L'huile de soja, la suppocire NC, le lipide amphiphile, le DOTAP, l'éventuel DOPE, ont été pesés puis mélangées avec du dichlorométhane avant d'être chauffés à 60°C afin d'obtenir une solution visqueuse homogène. Le dichlorométhane permet de favoriser la solubilisation. Les solvants ont ensuite évaporés sous vide.

### (ii) Préparation de la phase aqueuse :

Pendant la phase d'évaporation de l'éthanol, la phase aqueuse a été préparée. Dans un eppendorf de 5ml, le co-tensioactif, du glycérol et la solution aqueuse de PBS (NaCl 154 mM, pH 7,4) ont été mélangés puis dissous dans un bain à 75°C.

### (iii) Mélange des deux phases :

La phase huileuse était à environ 40°C (sous forme visqueuse) et la phase aqueuse à environ 70°C (en sortie du bain). La phase aqueuse a été versée dans la phase huileuse.

### (iv) Emulsification :

Le flacon contenant les deux phases a été fixé dans l'enceinte de sonication d'un sonificateur AV505® (Sonics, Newton, USA). Le protocole consistait en la réalisation de cycles de sonication (10 secondes d'activité toutes les 30 secondes) à une puissance de 100 W sur une période de 40 minutes.

### (v) Purification :

Les gouttelettes ainsi produites ont ensuite été purifiées par dialyse (seuil de coupure : 12 kDa, contre du NaCI 154 mM, sur une nuit) pour éliminer les composants lipidiques non-intégrés aux LNP. Finalement, la formulation a été stérilisée par filtration sur membrane de cellulose.

### • Taille des gouttelettes des formulations et potentiel zéta

### - Influence de la composition de la formulation

Les résultats des tableaux 1 à 5 montrent qu'une diminution de la proportion en co-tensioactif (Myrj S40) conduit à une augmentation du diamètre des gouttelettes.

### - Evolution dans le temps

L'évolution de la taille des gouttelettes (Tableau 6) et du potentiel zéta (tableau 7) des formulations ont été mesurées à 40°C (stabilité accélérée). Les formulations étaient conservées à 40°C entre deux mesures.

**Tableau 6 : Evolution de la taille des gouttelettes et index de polydispersité (PDI) mesurés par diffusion quasi-élastique de la lumière en fonction du temps**

| Jours | **B1** | **B4** | **B5** | **B6** | **B10** | **C1** | **C2** |
|---|---|---|---|---|---|---|---|
| **0** | 58,27 | 98,87 | 60,03 | 87,93 | 91,53 | 157,43 | 171,4 |
| **7** | 60,52 | 97,12 | 58,96 | 87,1 | 90,51 | 156,17 | 172,63 |
| **14** | 62,59 | 98,36 | 58,28 | 86,25 | 89,87 | 156,46 | 170,15 |
| **21** | 59,41 | 97,81 | 59,23 | 85,78 | 90,3 | 153,03 | 162,2 |
| **28** | 61 | 97,15 | 60,36 | 87,61 | 90,96 | 153,9 | 161,47 |

**Tableau 7 : Evolution du potentiel zéta des formulations en fonction du temps**

| Jours | **B1** | **B4** | **B5** | **B6** | **B10** | **C1** | **C2** |
|---|---|---|---|---|---|---|---|
| **0** | -21,3 | -21,16 | 24,03 | 34,13 | 34,97 | -40,27 | 57,33 |
| **7** | -25,6 | -21,17 | 28,23 | 31,77 | 34,73 | -43,13 | 56,77 |
| **14** | -24,73 | -21,03 | 29,45 | 28,4 | 33,47 | -42,33 | 55,33 |
| **21** | -21,8 | -20,47 | 28,63 | 34,3 | 33,07 | -38,1 | 53,9 |
| **28** | -18,93 | -19,83 | 27,5 | 33 | 31,23 | -39,03 | 51,6 |

Il a été observé que la taille des gouttelettes et le potentiel zéta de formulations selon l'invention conservées à 40°C pendant 300 jours n'évoluent pas.

Ces résultats montrent que les formulations selon l'invention sont stables dans le temps.

### 1.2. Complexation avec des siARN

La procédure générale suivante a été suivie :

La complexation consiste en un simple mélange des formulations préparées ci-dessus et d'une solution de siARN, le tout dans un tampon. Le choix du tampon est fonction de l'application envisagée : pour une étude *in vitro,* le milieu de culture optimisé pour les étapes de transfection, OptiMEM, a été utilisé. Pour une étude de complexation, du tampon Hepes 5 mM a été utilisé.

Une quantité de 0,5 µg de siARN (GFP-22 siRNA rhodamine (catalogue n°1022176) (Qiagen) ou siGFP (Sigma)) a été utilisée (25 µg/mL dans 20 µL).

Le mélange a été agité durant 30 minutes à 600 rpm, ceci à température ambiante (environ 25°C).

La complexation a été visualisée à travers deux outils :
- L'électrophorèse sur gel d'agarose qui permet d'observer la migration des siARN. S'il y a une bonne complexation, alors les gouttelettes comprenant les siARN complexés seront plus lourdes que les siARN libres et seront visualisés dans les puits. Si la complexation est moins importante, des siARN libres migrent à une autre position.
- En DLS en observant l'impact de la complexation sur le diamètre hydrodynamique. Plus la complexation est efficace, plus le profil s'oriente vers une distribution monomodale.

La quantité de formulation nécessaire pour avoir un rendement de complexation quantitatif des siARN a été optimisée.

En pratique, les charges négatives apportées par les siARN sont compensées par les charges positives de la formulation (c'est-à-dire les charges positives du tensioactif cationiques DOTAP).

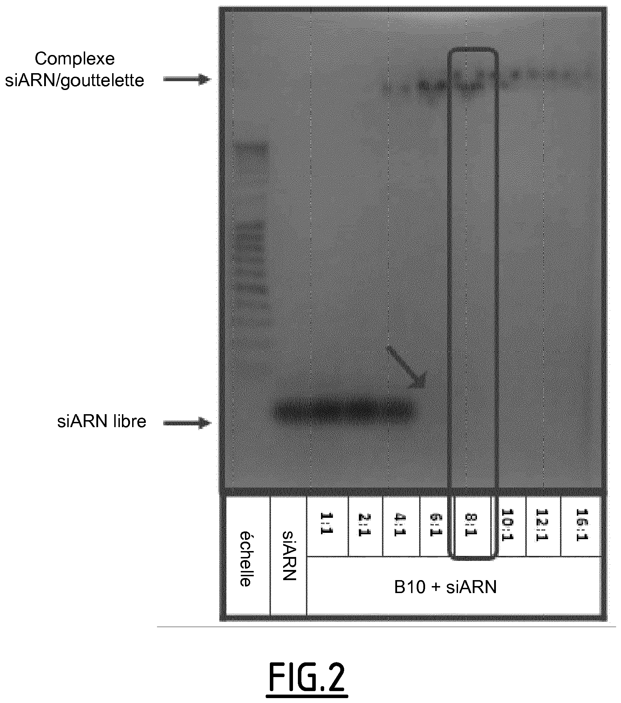

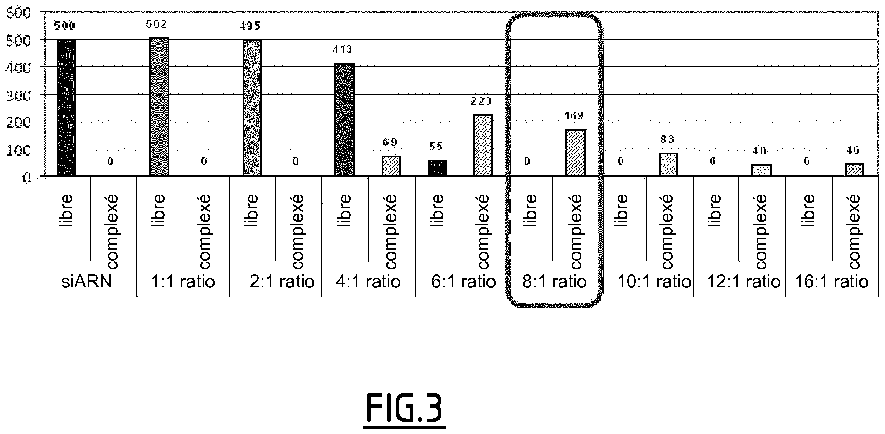

Typiquement, lorsque le seul tensioactif cationique de la formulation est du DOTAP (qui ne comprend qu'une seule charge positive), un rendement quantitatif de complexation est obtenu lorsque le rapport quantité de charges positives dues au tensioactif cationique dans la formulation « prémix » sur la quantité de charge négative apportée par les siARN est supérieur à 8/1, comme illustré sur les figures 2 et 3.

La figure 2 représente un gel d'électrophorèse révélé à l'UV réalisé après complexation de siARN avec la formulation B10 avec des concentrations précisées au tableau 8, en mélangeant une solution de siARN et la formulation dans un tampon Hepes 5 mM. Avant dépôt sur gel d'agarose 1,5 %, 2 µL de tampon de charge ont été ajoutés aux essais. Après 1h30 d'électrophorèse à 100 V, le gel a été plongé dans du GelRed 3X. Enfin, une révélation UV a été effectuée.

**Tableau 8**

| | | | | | | | | | |
|---|---|---|---|---|---|---|---|---|---|
| | rapport quantité de charges positives dues au tensioactif cationique dans la formulation « prémix » sur quantité de charge négative apportée par les siARN | 1/1 | 2/1 | 4/1 | 6/1 | 8/1 | 10/1 | 12/1 | 16/1 |
| Concentration en siARN (µg/mL) | 25 | | | | | | | | |
| Concentration en DOTAP (µg/mL) | 0 | 25 | 50 | 100 | 150 | 200 | 250 | 300 | 400 |

La figure 3 donne les résultats obtenus par traitement des données de l'électrophorèse sur le logiciel ImageJ des mêmes expériences.

Les figures 2 et 3 montrent que pour un rapport quantité de charges positives dues au tensioactif cationique dans la formulation « prémix » sur la quantité de charge négative apportée par les siARN supérieur à 8/1, il n'y a plus de siARN libre dans le milieu et que le siARN a été complètement complexé.

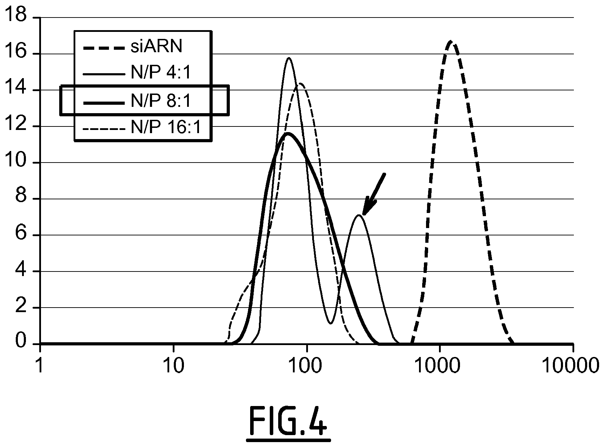

La figure 4 représente l'intensité obtenu sur un appareil ZetaSizer Malvern en fonction du diamètre hydrodynamique en nm pour le siARN libre (comparaison) et pour trois formulations selon l'invention obtenues par complexation avec un rapport quantité de charges positives dues au tensioactif cationique dans la formulation « prémix » sur la quantité de charge négative apportée par les siARN de 4/1, de 8/1 ou de 16/1.

Le diamètre est plus important pour le siARN libre (comparaison).

Avec un rapport quantité de charges positives dues au tensioactif cationique dans la formulation « prémix » sur la quantité de charge négative apportée par les siARN de 4/1, on a observé 2 populations : un complexe siARN/gouttelette autour de 100 nm et une population de taille supérieure représentant les siARN sous forme libre (flèche).

Avec les rapports quantité de charges positives dues au tensioactif cationique dans la formulation « prémix » sur la quantité de charge négative apportée par les siARN de 8/1 et 16/1, une seule population représentant le complexe siARN/ gouttelette a été observée.

Ces résultats montrent également qu'une complexation quantitative (100%) des siARN sur les gouttelettes est permise.

L'étape de complexation a été réalisée avec diverses formulations.

A titre comparatif, un test de complexation a été réalisé avec une formulation exempte de tensioactif cationique : la formulation B1 décrite ci-dessus. Comme attendu, la complexation du siARN n'a pas eu lieu et le siARN est resté sous forme libre.

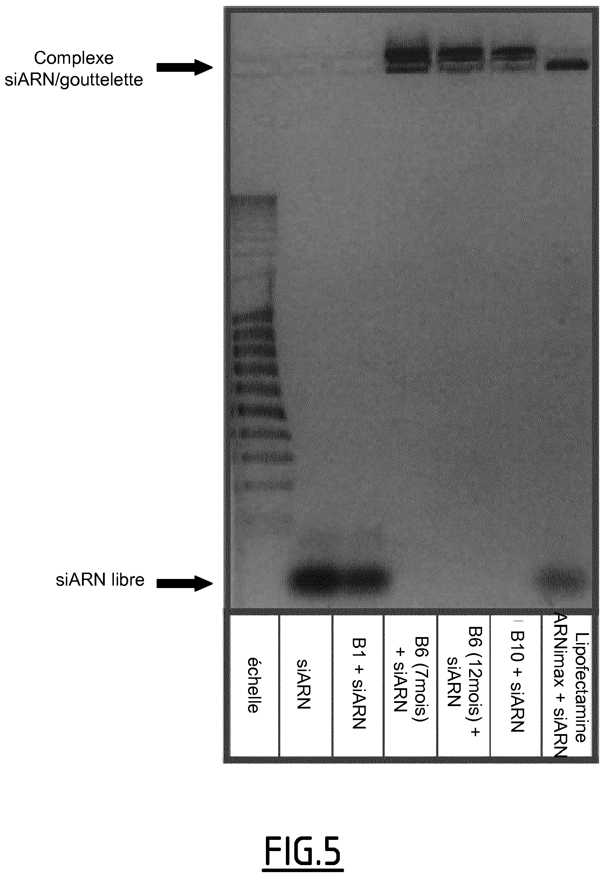

La figure 5 représente un gel d'électrophorèse révélé à l'UV avec, de gauche à droite :
- l'échelle,
- le siARN libre (témoin)
   puis des migrations obtenues par complexation de siARN :
- avec la formulation B1 (exemple comparatif) (pas de complexation, les siARN sont libres),
- avec la formulation B6 (prémix formulé 6 mois auparavant) avec un rapport quantité de charges positives dues au tensioactif cationique dans la formulation « prémix » sur la quantité de charge négative apportée par les siARN de 8/1 (complexation quantitative),
- avec la formulation B6 (prémix formulé 12 mois auparavant) avec un rapport quantité de charges positives dues au tensioactif cationique dans la formulation « prémix » sur la quantité de charge négative apportée par les siARN de 8/1, la formulation ayant été conservée 7 mois à température ambiante préalablement à la complexation (complexation quantitative),
- avec la formulation B10 avec un rapport quantité de charges positives dues au tensioactif cationique dans la formulation « prémix » sur la quantité de charge négative apportée par les siARN de 8/1 (complexation quantitative),
- avec de la lipofectamine (exemple comparatif) (complexation incomplète).

La complexation de siARN est quantitative lorsque la formulation B6 ou B10 ont été utilisées. Le stockage de la formulation à température ambiante avant sa complexation avec le siARN n'a pas d'influence sur le rendement de complexation, qui reste quantitatif, ce qui montre la stabilité des formulations utilisées.

Le rendement est quantitatif que la formulation utilisée comprennent ou non du DOPE.

Avec l'agent de transfection commercial Lipofectamine RNAimax, plus de 60 % des siARN ont été retrouvés sous forme libre. Ceci implique que les formulations de nanoémulsions utilisées dans l'invention offrent un meilleur rendement de complexation que la Lipofectamine.

Enfin, une cinétique de relargage des siARN du complexe siARN/formulation B10 préparé ci-dessus a été effectuée pour observer l'évolution de la complexation au cours du temps et est illustrée en figure 6. Jusqu'à 3 heures après complexation, les siARN ne sont pas retrouvés sous forme libre. A 6 heures, les siARN commencent à être relargués. Le complexe est donc stable au moins trois heures.

### 1.3. Transfection in vitro

Des tests de transfection préalables des nanoparticules définies dans l'exemple 1.1. et complexées selon l'exemple 1.2. avec un siARN inhibant spécifiquement une séquence de l'ARN messager de la GFP (GFP-22 siRNA rhodamine (catalog n°1022176) (Qiagen)) ont été effectués sur différentes lignées cellulaires sur-exprimant la Green Fluorescent Protein (GFP, Protéine de fluorescence verte).

La transfection a été effectuée avec une concentration finale en siARN de 100 nM. Ainsi, des cellules exprimant la GFP ont été ensemencées dans des plaques 12 puits (25 000 cellules/puits) puits traitées avec les complexes siARN/Formulations (B1, B6, B6 7 mois après sa préparation ou B10) obtenus ci-dessus. Les cellules sont ensuite incubées 72 heures à 37 °C, puis récupérées pour analyse de l'intensité de fluorescence au cytomètre en flux pour déterminer l'efficacité de la formulation en tant qu'agent de transfection. La délivrance active de siARN inhibant spécifiquement l'expression de la protéine GFP induit une diminution de la fluorescence apportée par cette protéine.

L'agent de transfection commercial, la Lipofectamine RNAimax a été utilisé pour comparaison.

La figure 7 représente la diminution de l'intensité de fluorescence FITC en % en transfectant les lignées cellulaires avec :
- de la Lipofectamine RNAimax,
- le complexe siARN/formulation B1,
- avec le complexe siARN/formulation B6, la formulation ayant été conservée 7 mois à température ambiante préalablement à la complexation,
- avec le complexe siARN/formulation B6, la formulation ayant été conservée 12 mois à température ambiante préalablement à la complexation,
- avec le complexe siARN/formulation B10,
- avec un complexe siARN / formulation de liposomes cationiques comprenant du DOTAP (58% wt), du DOPE (18% wt), du cholestérol (2% wt) et du DSPE-PEG3000 (22% wt)) (comparatif).

Ainsi, une diminution de la fluorescence de 33 à 50% est observée avec les formulations selon l'invention testées. Les formulations selon l'invention permettent donc une délivrance active de siARN induisant une extinction relative de l'expression du gène de la GFP.

De plus, en incorporant du DOPE dans les formulations, une plus importante extinction de fluorescence est visible, le DOPE favorisant l'échappement endosomal.

Aucune diminution de la fluorescence n'a été observée avec le complexe siARN / formulation de liposomes cationiques, ce qui pourrait par exemple s'expliquer par une mauvaise stabilité des liposomes dans le milieu de culture, un mauvais rendement de complexation et/ou une mauvaise rétention des siARN par les liposomes après complexation.

Enfin, de tels résultats sur la délivrance active de siARN médiée par les formulations selon l'invention ont été reproduits sur 3 lignées cellulaires exprimant la GFP : U2OS, PC3 et Hela, comme illustré en figure 8.

### 1.4. Complexation avec les étiquettes ADN uniques

La complexation consiste en un simple mélange des formulations préparées ci-dessus et d'une solution de siARN, le tout dans un tampon. Le choix du tampon est fonction de l'application envisagée : pour une étude *in vitro,* le milieu de culture optimisé pour les étapes de transfection, OptiMEM, a été utilisé. Pour une étude de complexation, du tampon Hepes 5 mM a été utilisé. Le mélange a été agité durant au moins 30 minutes à 600 rpm, ceci à température ambiante (environ 25°C).

### 1.5. Encapsulation du fluorophore

L'encapsulation du fluorophore est réalisée selon la méthode définie dans la demande WO2008104717. Plus précisément, es nanoparticules fluorescentes sont obtenues par sonication de la phase huileuse dans la phase aqueuse. La phase huileuse comprend un mélange d'huile de soja et de Suppocire^{®} NC ainsi que la lécithine et les fluorophores (pour des raisons de solubilité). La phase aqueuse comprend quand à elle, le surfactant pegylé, la solution aqueuse (NaCl ou PBS) et éventuellement du glycérol afin d'augmenter la viscosité du mélange. Les nanoparticules fluorescentes sont produites par lot de 2 ou 5 mL. Brièvement, la phase huileuse est préparée par mélange de l'huile de soja, de la Suppocire^{®} NC et de la lécithine (phase dispersée + lécithine). Un solvant organique (dichlorométhane) est ajouté pour faciliter la dissolution de la lécithine. Une fois tous les composés dissous, le solvant est évaporé sous vide à une température supérieure à celle de fusion de la cire. Les molécules fluorescentes sont ensuite ajoutées dans la phase huileuse. Pour faciliter leur dispersion, les fluorophores sont préalablement dissous dans un solvant organique (éthanol). La solution est homogénéisée et le solvant organique est retiré par évaporation sous vide. La phase aqueuse est préparée par mélange à chaud du glycérol, du surfactant pegylé et de la solution aqueuse. Les deux phases préalablement maintenues à environ 50°C sont mélangées puis homogénéisées par ultrasons, de manière à former des nanoparticules piégeant en leurs coeurs les fluorophores lipophiles. Les solutions de nanoparticules fluorescentes obtenues sont ensuite purifiées par dialyse de manière à éliminer les molécules n'ayant éventuellement pas été encapsulées.

### 1.6. Préparation de la bibliothèque de nanoparticules

La préparation de 1000 nanoparticules fluorescentes est réalisée en utilisant des siARN provenant de la collection de 1292 siARN de Qiagen ciblant 646 kinases (Human Kinase siRNA set V1.0 ; Ref 1027091), ou la collection de 2375 siARN de Qiagen ciblant 1183 gènes impliqués dans les cancers (Human Cancer siRNA set V2.0), ou la collection de 278 siARN ciblant 139 gènes impliqués dans les cancers (Human Cancer siRNA set V1.0; Ref 1022171), ou la collection de 91800 siARN de Qiagen ciblant 22950 gènes humains (Human Genome Wide siRNA set), ou en utilisant des LNA provenant de la collection de 982 LNA d'Exiqon ciblant tous les miARN humains connus (miRCURY LNA Human microRNA Inhibitor Library; Ref 190102-2).

L'étiquette ADN unique contient deux séquences de 50 pb en 5' et en 3' permettant l'amplification de l'étiquette flanquant une séquence unique de 10 pb contenant des bases synthétiques. Chaque siARN ou chaque LNA des collections est associé *in silico* avec une séquence unique de 10 pb.

### Exemple 2 : Culture en 2D et en 3D de cellules prostatiques

Les cellules prostatiques suivantes sont utilisées :
- les cellules PTN1 et RWPE1, qui sont des cellules épithéliales normales de prostate immortalisées,
- les cellules WPE1-NA22, WPE1-NB14, WPE1-NB11 et WPE1-NB26, qui sont dérivées des cellules RWPE1 et qui miment différents stades de tumorigénèse après exposition au N-Méthyl-N-Nitrosourée,
- la lignée cellulaire 22Rv1, qui est une lignée de cellules épithéliales de carcinome prostatique humain répondant à la privation en androgènes,
- les lignées cellulaires VcaP et LNCaP, qui sont des lignées de cellules cancéreuses prostatiques métastatiques sensibles *in vitro* et *in vivo* aux androgènes,
- les lignées cellulaires PC3 et DU145, qui sont des lignées de cellules cancéreuses prostatiques métastatiques qui ne répondant plus à la privation en androgènes,
- des cellules primaires prostatiques issues de sujets sains.

La production de structures 3D en forme d'acini à partir de ces cellules prostatiques est également réalisée.

L'efficacité de transfection et la toxicité des nanoparticules comprenant un fluorophore, un siARN et une étiquette ADN unique sont évaluées sur chacune de ces cultures de cellules prostatiques.

### Exemple 3 : Analyse phénotypique à haut-débit par cytométrie en flux

Les cellules sont criblées selon deux phénotypes différents : la prolifération cellulaire, la mort cellulaire. La prolifération est évaluée par marquage à l'iodure de propidium, à l'EdU, ou à l'Hoechst 33342. La mort cellulaire est évaluée par marquage Annexine V-FITC ou par marquage dit TUNEL (« Terminal deoxynucleotidyl transferase dUTP Nick End Labeling » en anglais) ou par l'analyse de l'activation des caspases. Pour chacun de ces phénotypes, les conditions optimales de marquage et de sélection des cellules sont évaluées, afin de sélectionner les contrôles positifs et négatifs appropriés.

Une fois les conditions de marquage et de sélection des cellules selon leur phénotype déterminées, des criblages primaires sur environ 10 lignées cellulaires prostatiques présentant différents types de réponse à un traitement hormonal ou sur des cellules primaires prostatiques sont réalisés.

### Exemple 4 : Déconvolution de l'étiquette ADN unique, analyse des données et validation

Un petit aliquot des cellules sélectionnées après cytométrie en flux est utilisé pour identifier l'étiquette ADN unique. L'ADN est extrait des cellules fluorescentes, soumis à une PCR avec des amorces universelles, et séquencé avec un séquenceur de seconde génération (Illumina ou Roche 454 ou ABI solid). L'identification de la séquence de 10 pb de l'étiquette ADN unique est ensuite corrélé avec le siARN ou le LNA qui lui a été associé *in silico.* Une liste de gènes dont l'inhibition par siARN ou LNA induit le phénotype d'intérêt est ensuite établie. La méthode de criblage permet également d'établir des ensembles de données génomiques fonctionnelles pour chacune des lignées cellulaires testées en fonction de leur sensibilité à des thérapies hormonales, des listes de gènes codant des protéines ou des miARN qui sont des marqueurs potentiels de la sensibilité aux hormones.

Une validation *in vitro* sur les cellules restantes de la survenue du phénotype et de l'inhibition du gène est réalisée par PCR quantitative en temps réel et par Western-blot.

### Exemple 5 : Validations in vivo et validations cliniques

Une stratégie de validation à deux temps est réalisée :
- pour la validation *in vivo,* des cellules prostatiques humaines cancéreuses contrôles et des cellules cancéreuses prostatiques transfectées avec les nanoparticules selon l'invention (e.g. contenant des siARN ou des LNA) sont implantées dans des cellules « nude » athymiques. Le volume de tumeurs est surveillé et, après euthanasie, les tumeurs sont excisées et fixées pour l'analyse immunohistologique de la prolifération ou de l'apoptose dans les cellules transformées avec les nanoparticules selon l'invention.
- pour la validation clinique, des micropuces commerciales de tissus prostatiques contenant plusieurs centaines de tissus prostatiques cancéreux de différents grades sont utilisées.

Ceci permet la validation de biomarqueurs du cancer de la prostate, de nouvelles cibles thérapeutiques et de siARN en tant que nouveaux agent thérapeutiques.

### Exemple 6 : Co-délivrance de siARN d'intérêt et d'une étiquette ADN (code barre) par des nanoparticules fluorescentes, tri des cellules avant incorporé les nanoparticules selon un phénotype d'intérêt et identification a posteriori du code barre de l'étiquette ADN par extraction d'ADN et amplification spécifique à partir des cellules triées

### 6.1. Complexation entre différents acides nucléiques_{.} ADN étiquette et siARN_{.} avec une formulation de nanoparticules fluorescentes

Dans cet exemple une co-transfection des cellules cibles est réalisée avec un siARN d'intérêt et une étiquette ADN spécifique du siRNA en ayant recours à une dispersion de nanoparticules fluorescentes.

### Expérience de retard sur gel (Figure 9):

Les formulations de nanoparticules lipidiques utilisées dans cet exemple ont été réalisées selon le même procédé de fabrication que celui décrit dans l'exemple 1 et correspondent à la formulation A3. La procédure générale de complexation est la même que celle suivie pour l'exemple 1. Brièvement, la complexation consiste en un simple mélange d'une formulation A3 comprenant un fluorophore lipophile encapsulé dans le coeur (DiD, Invitrogen, Ref D7757) et d'une solution de siARN et d'étiquette ADN avec les nanoparticules, le tout dans un tampon. Dans cette étude, le tampon utilisé est de l'HEPES (5 mM, pH 7,2). Pour le puits 1, les nanoparticules A3 ont été complexées avec une solution contenant 11 ng d'un siARN (siAllStar Negative Control siRNA, Qiagen, Ref 1027280) et 20 ng d'étiquette ADN (Eurogentec). Pour les puits suivants (puits numérotés de 2 à 7), cette solution siARN/étiquette ADN a été diluée par deux avant complexation avec les nanoparticules, selon la technique de dilution en cascade. Les ratios N/P utilisé ici (N = charge positive apportée par le groupement ammonium de l'azote des lipides cationiques composant la couronne de la particule lipidique ; P = charge négative apportée par le groupement phosphate des acides nucléiques) sont respectivement de 12/1, 24/1, 48/1, 96/1, 192/1, 384/1, 768/1 pour les puits 1 à 7. Le mélange a été agité durant 30 minutes à 600 rpm à température ambiante (environ 25°C). Une électrophorèse sur gel d'agarose (gel à 1,5% d'agarose avec Agarose ultrapure 1000, Invitrogen, Ref 16550100; tampon TBE 10X, Ref 15581044; Ultrapure water, Ref 10977035) permet de démontrer que les deux acides nucléiques sont bien complexés au niveau de la gouttelette lipidique fluorescente, tel qu'illustré sur la figure 9. En effet, si les complexes nanoparticules/siARN/étiquette ADN sont stables, tous les acides nucléiques (siARN et étiquette ADN) sont retenus dans les puits (puits 1 à 7) et ne peuvent pas migrer à l'intérieur du gel. A l'inverse, un siARN libre (non complexé avec les nanoparticules) sera visible sous forme d'une bande migrant vers 21 pb (puits 8). De même, une étiquette ADN libre (non complexée avec les nanoparticules) migrera sous forme d'une bande visible vers 129 pb (puits 9). Cette expérience montre l'efficacité des nanoparticules lipidiques, type Lipidot^{®} de formulation A3, à complexer simultanément et de manière stable des siARN et des étiquettes ADN.

### 6.2. Co-transfection de deux acides nucléiques, siARN et étiquette ADN, par des nanoparticules fluorescentes

### Expérience de transfection in vitro de HeLa sur-exprimant la GFP avec des complexes formulation A3/ étiquette ADN /siARN:

La délivrance simultanée, dans des cellules cibles *in vitro,* des différentes molécules transportées par la nanoparticule lipidique (formulation A3) contenant un fluorophore a été évaluée.

Pour se faire, une étude a été effectuée sur des cellules HeLa, i.e. des cellules issues du cancer du col de l'utérus (ATCC, Ref HeLa-CCL2), qui ont été modifiées afin de surexprimer la protéine GFP. La délivrance active de siARN est validée par l'extinction de la protéine GFP (diminution du signal FITC) grâce à un ciblage spécifique de son ARNm par un siGFP. En parallèle, l'observation de l'interaction des nanoparticules fluorescentes avec la cellule est rendue possible par l'étude de l'évolution du signal fluorescent apporté par le fluorophore encapsulé, le DID (suivi du signal APC). Cette première étape montre une très forte diminution du signal FITC et donc de l'expression de la GFP dans les cellules traitées avec le complexe nanoparticule A3/étiquette ADN /siGFP ainsi qu'une augmentation du signal APC témoignant de l'interaction des nanoparticules fluorescentes (porteuses du fluorophore DID) avec les cellules (figures 10 et 11).

Ces résultats montrent que la présence de l'ADN étiquette ne perturbe pas la délivrance du siARN dans les cellules et que la fonctionnalité de ce dernier est préservée, à savoir l'interaction avec l'ARNm codant pour la GFP et l'inhibition de l'expression de cette protéine par mécanisme d'interférence ARN. L'efficacité d'extinction de la fluorescence FITC correspondant à l'expression de la GFP est de l'ordre de 70% dans ces expériences suite à l'incubation des cellules avec les complexes nanoparticules fluorescentes A3/étiquette ADN / siARN ciblé contre l'ARNm de la GFP (Figure 10).

### Identification de l'étiquette d'ADN dans les cellules triées sur la base de l'extinction de la GFP après transfection par des complexes A3 /étiquette ADN /siARN:

Dans cette expérience, les populations cellulaires exprimant fortement ou faiblement la GFP ont été triées à l'aide d'un cytomètre en flux (Cytomation, MoFlo). En pratique, 200 000 cellules HeLa-GFP ont été ensemencées dans des puits de plaque 6 puits. Après 24 heures de culture, ces cellules ont été transfectées avec des complexes nanoparticules fluorescentes A3/étiquette ADN/siAllStar ou des complexes nanoparticules fluorescentes A3/étiquette ADN/siGFP. Soixante-douze heures après transfection, les cellules sont décollées des puits par addition de trypsine. Les cellules contenues dans trois puits pour une même condition de transfection sont additionnées et triées en deux populations distinctes, selon leur niveau d'expression fort ou faible de la GFP (Figure 12). Le contenu en ADN des cellules ainsi récupérées est extrait *via* le kit QiaAmp DNA mini kit (Qiagen, Ref 51304) puis une PCR est réalisée en utilisant les amorces spécifiques de l'étiquette ADN. Les amorces utilisées pour cette amplification sont les primers pGEX 3' et 5' (Eurogentec, Ref UN-PR130-005 et UN-PR135-005). La PCR est effectuée avec 35 cycles d'amplification (Qiagen, HotStarTaq Master Mix, Ref 203443). Les acides nucléiques ainsi amplifiés sont ensuite déposés dans les puits d'un gel d'agarose 2% préalablement préparé. Dans la figure 13, nous pouvons observer que les cellules transfectées avec les complexes nanoparticules fluorescentes A3/étiquette ADN/siGFP présentent une très forte diminution de l'expression de la GFP, et sont également porteuse de l'étiquette ADN qui est détectée par PCR. Ce résultat montre la capacité des nanoparticules fluorescentes de formulation A3 à délivrer simultanément au sein des cellules, une étiquette ADN et un siRNA fonctionnel.

Ces résultats démontrent :
- que les nanoparticules selon l'invention permettent la délivrance simultanée dans des cellules cibles de deux acides nucléiques (siARN et étiquette ADN, les cellules transfectées par le siARN étant donc fluorescente.
- la faisabilité du tri des cellules ayant incorporées les nanoparticules fluorescentes et présentant un phénotype d'intérêt,
- la faisabilité de l'identification a posteriori du siARN d'intérêt par analyse du code barre de l'étiquette ADN spécifiquement associée à ce siARN, après extraction de l'ADN cellulaire et amplification de l'ADN étiquette.

## Revendications

1. Méthode de criblage d'une molécule d'intérêt comprenant les étapes suivantes :
a) mettre en contact des cellules avec une bibliothèque de nanoparticules synthétiques, chaque nanoparticule synthétique comprenant une molécule candidate, un traceur et une étiquette ADN unique propre à ladite molécule candidate, dans des conditions permettant auxdites cellules d'intégrer au moins une desdites nanoparticules synthétiques, la molécule candidate étant située à la surface de la nanoparticule synthétique,
b) sélectionner les cellules ayant intégré le traceur et présentant un phénotype d'intérêt,
c) identifier en tant que molécule d'intérêt la molécule candidate ayant été intégrée dans la cellule sélectionnée à l'étape b) en identifiant la séquence de ladite étiquette ADN unique.

2. Méthode de criblage selon la revendication 1, dans laquelle le traceur est un traceur magnétique et la sélection des cellules ayant intégré ledit traceur magnétique est réalisée par tri cellulaire magnétique, ou le traceur est un fluorophore et la sélection des cellules ayant intégré ledit fluorophore est réalisée par cytométrie en flux.

3. Méthode de criblage selon la revendication 1 ou 2, dans laquelle chaque nanoparticule est une particule de moins de 1 micron de diamètre.

4. Méthode de criblage selon l'une quelconque des revendications 1 à 3, dans laquelle lesdites nanoparticules comprennent en outre un ligand biologique de ciblage d'une cellule et/ou d'un organe.

5. Méthode de criblage selon l'une quelconque des revendications 1 à 4, dans laquelle ladite nanoparticule est une nanoparticule inorganique, une nanoparticule organique, ou une nanoparticule hybride.

6. Méthode de criblage selon la revendication 5, dans laquelle ladite nanoparticule est un vecteur lipidique.

7. Méthode de criblage selon la revendication 6, dans laquelle ledit vecteur lipidique est une gouttelette d'une formulation sous forme d'une nano-émulsion, comprenant une phase aqueuse continue et au moins une phase dispersée, et comprenant :
a) au moins 5% molaire de lipide amphiphile,
b) de 15 à 70% molaire d'au moins un tensioactif cationique comprenant :
i) au moins un groupe lipophile choisi parmi :
- un groupe R ou R-(C=O)-, où R représente une chaîne hydrocarbonée linéaire comprenant de 11 à 23 atomes de carbone,
- un ester ou un amide d'acides gras comprenant de 12 à 24 atomes de carbone et de phosphatidyléthanolamine, et
- un poly(oxyde de propylène), et
ii) au moins un groupe hydrophile comprenant au moins un groupe cationique choisi parmi :
- un groupe alkyle linéaire ou ramifié comprenant de 1 à 12 atomes de carbone et interrompu et/ou substitué par au moins un groupe cationique, et
- un groupe polymérique hydrophile comprenant au moins un groupe cationique, et
c) de 10% à 55% molaire d'un co-tensioactif comprenant au moins une chaîne poly(oxyde d'éthylène) comprenant au moins 25 unités d'oxyde d'éthylène,
d) un lipide solubilisant,
e) éventuellement un lipide fusogène,
où les pourcentages molaires de lipide amphiphile, de tensioactif cationique et de co-tensioactif sont par rapport à l'ensemble (lipide amphiphile / tensioactif cationique / co-tensioactif / éventuel lipide fusogène).

8. Méthode de criblage selon la revendication 7, dans laquelle dans ladite nano-émulsion :
- ledit tensioactif cationique est choisi parmi :
- le *N*[1-(2,3-dioléyloxy) propyl]-*N*,*N*,*N-*triméthylammonium,
- le 1,2-dioleyl-3-trimethylamonium-propane,
- le *N-*(2-hydroxyethyl)-*N*,*N-*dimethyl-2,3-bis(tetradecyloxy-1-propananium),
- le 1-[2-(oleoyloxy)ethyl]-2-oleyl-3-(2-hydroxyethyl)imidazolinium, et
- le dioctadecylamidoglycylspermine ; et/ou
- ledit lipide fusogène est la 1,2-Dioléoyl-*sn*-Glycéro-3-Phosphoéthanolamine ; et/ou
- ledit lipide amphiphile est un phospholipide.

9. Méthode de criblage selon la revendication 7 ou 8, dans laquelle, dans ladite nano-émulsion, ledit co-tensioactif est greffé avec ledit ligand biologique de ciblage d'une cellule et/ou d'un organe.

10. Méthode de criblage selon l'une quelconque des revendications 1 à 9, dans laquelle ladite molécule candidate est une séquence nucléotidique susceptible de moduler des mécanismes d'interférence par ARN.

11. Méthode de criblage selon la revendication 10, dans laquelle ladite séquence nucléotidique susceptible de moduler des mécanismes d'interférence par ARN est :
(i) un petit ARN interférent (siARN),
(ii) un acide nucléique bloqué (LNA), ou
(iii) un microARN (miARN),
(iv) un ARN long double brin (ARNdb).

12. Méthode de criblage selon l'une quelconque des revendications 1 à 11, dans laquelle ladite étiquette ADN unique consiste en une séquence d'ADN, de préférence double brin, d'au moins 50 nucléotides ou paires de bases (pb) consistant en, sur un brin et dans le sens 5'-3' :
- une première séquence d'au moins 20 nucléotides commune à toutes les étiquettes ADN uniques de la bibliothèque,
- une séquence unique d'au moins 10 nucléotides propre à ladite molécule,
- une deuxième séquence d'au moins 20 nucléotides commune à toutes les étiquettes ADN uniques de la bibliothèque.

13. Méthode de criblage d'un biomarqueur d'une maladie et/ou d'un trait phénotypique comprenant les étapes suivantes :
a) mettre en contact des cellules avec une bibliothèque de nanoparticules synthétiques, chaque nanoparticule étant telle que définie dans l'une quelconque des revendications 1 à 12, dans des conditions permettant auxdites cellules d'intégrer au moins une desdites nanoparticules,
b) sélectionner les cellules ayant intégré le traceur et présentant un phénotype d'intérêt,
c) identifier la molécule candidate ayant été intégrée dans la cellule sélectionnée à l'étape b) en identifiant la séquence de ladite étiquette ADN unique,
d) identifier en tant que biomarqueur d'une maladie et/ou d'un trait phénotypique la cible de ladite molécule identifiée à l'étape c).

14. Méthode selon la revendication 13, dans laquelle ladite maladie est un cancer ou une infection.

15. Utilisation de nanoparticules synthétiques telles que définies dans l'une quelconque des revendications 1 à 12 pour le criblage de molécules d'intérêt et/ou de biomarqueur d'une maladie et/ou d'un trait phénotypique.

16. Bibliothèque de nanoparticules synthétiques telles que définies dans l'une quelconque des revendications 1 à 12.

17. Nanoparticule synthétique telle que définie dans l'une quelconque des revendications 1 à 12.

18. Nanoparticule synthétique selon la revendication 17, dans laquelle ladite étiquette ADN unique consiste en une séquence d'ADN, de préférence double brin, d'au moins 50 nucléotides ou paires de bases (pb).

## Patentansprüche

1. Verfahren zum Screening eines interessierenden Moleküls, aufweisend die folgenden Schritte:
a) In-Kontakt-Bringen von Zellen mit einer Bibliothek von synthetischen Nanopartikeln, wobei jedes synthetische Nanopartikel ein zu prüfendes Molekül, einen Indikator und eine einzigartige Etiketten-DNA, die für das genannte zu prüfende Molekül spezifisch ist, aufweist, unter Bedingungen, die es den genannten Zellen erlauben, mindestens eines der genannten synthetischen Nanopartikel zu integrieren, wobei das zu prüfende Molekül an der Oberfläche des synthetischen Nanopartikels angeordnet ist;
b) Auswählen der Zellen, die den Indikator integriert haben und einen interessierenden Phänotyp darstellen;
c) Identifizieren des zu prüfenden Moleküls, das in der in Schritt b) ausgewählten Zelle integriert wurde, als das interessierende Molekül, indem die Sequenz der genannten einzigartigen Etiketten-DNA identifiziert wird.

2. Verfahren zum Screening gemäß Anspruch 1, wobei der Indikator ein magnetischer Indikator ist und die Auswahl der Zellen, die den genannten magnetischen Indikator integriert haben, mittels magnetischer Zellsortierung durchgeführt wird oder der Indikator ein Fluorophor ist und die Auswahl der Zellen, die das genannte Fluorophor integriert haben, mittels Durchflusszytometrie durchgeführt wird.

3. Verfahren zum Screening gemäß Anspruch 1 oder 2, wobei jedes Nanopartikel ein Partikel von weniger als 1 µm Durchmesser ist.

4. Verfahren zum Screening gemäß irgendeinem der Ansprüche 1 bis 3, wobei die genannten Nanopartikel außerdem einen biologischen Liganden zum Abzielen auf eine Zelle und/oder ein Organ aufweisen.

5. Verfahren zum Screening gemäß irgendeinem der Ansprüche 1 bis 4, wobei das genannte Nanopartikel ein anorganisches Nanopartikel, ein organisches Nanopartikel oder ein Hybrid-Nanopartikel ist.

6. Verfahren zum Screening gemäß Anspruch 5, wobei das genannte Nanopartikel ein Lipid-Vektor ist.

7. Verfahren zum Screening gemäß Anspruch 6, wobei der genannte Lipid-Vektor ein Tröpfchen einer Rezeptur in Form von einer Nano-Emulsion ist, die eine kontinuierliche wässrige Phase und mindestens eine dispergierte Phase aufweist, und aufweist:
a) mindestens 5 Mol-% amphiphile Lipide,
b) 15 bis 70 Mol-% mindestens eines kationischen Tensids, aufweisend:
i) mindestens eine lipophile Gruppe, ausgewählt aus:
- einer Gruppe R oder R-(C=O)-, wobei R eine lineare Kohlenwasserstoffkette ist, die 11 bis 23 Kohlenstoffatome aufweist,
- ein Ester oder ein Fettsäure-Amid, das 12 bis 24 Kohlenstoffatome und Phosphatidylethanolamin aufweist, und
- ein Poly(Propylenoxid), und
ii) mindestens eine hydrophile Gruppe, die mindestens eine kationische Gruppe aufweist, ausgewählt aus:
- einer linearen oder verzweigten Alkylgruppe, die 1 bis 12 Kohlenstoffatome aufweist und unterbrochen und/oder durch mindestens eine kationische Gruppe substituiert ist, und
- eine hydrophile Polymergruppe, die mindestens eine kationische Gruppe aufweist, und
c) 10 bis 55 Mol-% eines Co-Tensids, das mindestens eine Kette Poly(Ethylenoxid) aufweist, die mindestens 25 Einheiten Ehtylenoxid aufweist,
d) ein Solubilisierungslipid,
e) eventuell ein fusogenes Lipid,
wobei die Mol-Prozentangaben des amphiphilen Lipids, des kationischen Tensids und des Co-Tensids sich auf die Gesamtheit (amphiphiles Lipid / kationisches Tensid / Co-Tensid / fusogenes Lipid) beziehen.

8. Verfahren zum Screening gemäß Anspruch 7, wobei in der genannten Nano-Emulsion:
- das genannte kationische Tensid ausgewählt ist aus:
- N[1-(2,3-Dioleyloxy)propyl]-N,N,N-Trimethylammonium,
- 1,2-Dioleyl-3-Trimethylammonium-Propan,
- N-(2-Hydroxyethyl)-N,N,-Dimethyl-2,3-bis(Tetradecyloxy-1-Propananium),
- 1-[2-(Oleoyloxy)Ethyl]-2-Oleyl-3-(2-Hydroxyethyl)Imidazol, und
- Dioctadecylamidoglycylspermin; und/oder
- das genannte fusogene Lipid 1,2-Dioleoyl-*sn*-Glycero-3-Phosphoethanolamin ist; und/oder
- das genannte amphiphile Lipid ein Phospholipid ist.

9. Verfahren zum Screening gemäß Anspruch 7 oder 8, wobei in der genannten Nano-Emulsion der genannte biologische Ligand zum Abzielen auf eine Zelle und/oder ein Organ auf das genannte Co-Tensid übertragen wird.

10. Verfahren zum Screening gemäß irgendeinem der Ansprüche 1 bis 9, wobei das genannte zu prüfende Molekül eine Nukleotid-Sequenz ist, die geeignet ist, RNA-Interferenzmechanismen anzupassen.

11. Verfahren zum Screening gemäß Anspruch 10, wobei die genannte Nukleotid-Sequenz, die geeignet ist zum Anpassen von RNA-Interferenzmechanismen, ist:
(i) eine small interfering RNA (siRNA),
(ii) eine locked nucleic acid (LNA), oder
(iii) eine microRNA (miRNA),
(iv) eine lange doppelsträngige RNA (dsRNA).

12. Verfahren zum Screening gemäße irgendeinem der Ansprüche 1 bis 11, wobei die genannte einzigartige Etiketten-DNA aus einer vorzugsweise doppelstrangigen DNA-Sequenz von mindestens 50 Nukleotiden oder Basenpaaren(bp) besteht, bestehend aus, auf einem Strang und in der 5'-3'-Richgung:
- einer ersten Sequenz von mindestens 20 Nukleotiden, die allen einzigartigen Etiketten-DNAs der Bibliothek gemeinsam ist,
- einer einzigartigen Sequenz von mindestens 10 Nukleotiden, die spezifisch für das genannte Molekül ist,
- eine zweite Sequenz von mindestens 20 Nukleotiden, die allen einzigartigen Etiketten-DNAs der Bibliothek gemeinsam ist.

13. Verfahren zum Screening eines Biomarkers einer Krankheit und/oder eines phänotypischen Merkmals, die folgenden Schritte aufweisend:
a) In-Kontakt-Bringen von Zellen mit einer Bibliothek von synthetischen Nanopartikeln, wobei jedes Nanopartikel ist, wie gemäß irgendeinem der Ansprüche 1 bis 12 definiert, unter Bedingungen, die es den genannten Zellen erlauben, mindestens eines der genannten Nanopartikel zu integrieren,
b) Auswählen der Zellen, die den Indikator integriert haben und einen interessierenden Phänotyp darstellen,
c) Identifizieren des zu prüfenden Moleküls, das in der in Schritt b) ausgewählten Zelle integriert wurde, indem die Sequenz der genannten einzigartigen Etiketten-DNA identifiziert wird,
d) Identifizieren der Zielgruppe des genannten, in Schritt c) identifizierten Moleküls als Biomarker einer Krankheit und/oder eines phänotypischen Merkmals.

14. Verfahren gemäß Anspruch 13, wobei die genannte Krankheit Krebs oder eine Infektion ist.

15. Verwenden der synthetischen Nanopartikel, wie in irgendeinem der Ansprüche 1 bis 12 definiert, für das Screening von interessierenden Molekülen und/oder von Biomarkern einer Krankheit und/oder eines phänotypischen Merkmals.

16. Bibliothek synthetischer Nanopartikel, wie in irgendeinem der Ansprüche 1 bis 12 definiert.

17. Synthetisches Nanopartikel, wie in irgendeinem der Ansprüche 1 bis 12 definiert.

18. Synthetisches Nanopartikel gemäß Anspruch 17, wobei die genannte einzigartige Etiketten-DNA aus einer vorzugsweise doppelstrangigen DNA-Sequenz von mindestens 50 Nukleotiden oder Basenpaaren (bp) besteht.

## Claims

1. A method for screening a molecule of interest comprising the following steps:
a) putting into contact cells with a library of synthetic nanoparticles, each synthetic nanoparticle comprising a candidate molecule, a tracer and a single DNA tag specific to said candidate molecule, under conditions allowing said cells to integrate at least one of said synthetic nanoparticles, the candidate molecule being localised at the surface of of the synthetic nanoparticle,
b) selecting the cells having integrated the tracer and having a phenotype of interest,
c) identifying as a molecule of interest, the candidate molecule having been integrated into the cell selected in step b) by identifying the sequence of said single DNA tag.

2. The screening method according to claim 1, wherein the tracer is a magnetic tracer and the selection of the cells having integrated said magnetic tracer is carried out by magnetic cell sorting or the tracer is a fluorophore and the selection of the cells having integrated said fluorophore is carried out by flow cytometry.

3. The screening method according to claim 1 or 2, wherein each nanoparticle is a particle with a diameter of less than 1 micron.

4. The screening method according to any of claims 1 to 3, wherein said nanoparticles further comprise a biological ligand for targeting a cell and/or an organ.

5. The screening method according to any of claims 1 to 4, wherein said nanoparticle is an inorganic nanoparticle, an organic nanoparticle, or a hybrid nanoparticle.

6. The screening method according to claim 5, wherein said nanoparticle is a lipid vector.

7. The screening method according to claim 6, wherein said lipid vector is a droplet of a formulation in the form of a nano-emulsion, comprising a continuous aqueous phase and at least one dispersed phase, and comprising:
a) at least 5% molar of an amphiphilic lipid,
b) from 15 to 70% molar of at least one cationic surfactant comprising:
i) at least one lipophilic group selected from:
- a group R or R-(C=O)-, wherein R represents a linear hydrocarbon chain comprising from 11 to 23 carbon atoms,
- an ester or an amide of fatty acids comprising from 12 to 24 carbon atoms and phosphatidylethanolamine, and
- a poly(propylene oxide), and
ii) at least one hydrophilic group comprising at least one cationic group selected from:
- a linear or branched alkyl group comprising from 1 to 12 carbon atoms and interrupted and/or substituted with at least one cationic group, and
- a hydrophilic polymeric group comprising at least one cationic group, and
c) from 10% to 55% molar of a co-surfactant comprising at least one poly(ethylene oxide) chain comprising at least 25 ethylene oxide units,
d) a solubilizing lipid,
e) optionally a fusogenic lipid,
wherein the molar percentages of amphiphilic lipid, of cationic surfactant and of co-surfactant are relatively to the (amphiphilic lipid/cationic surfactant/co-surfactant/optional fusogenic lipid) assembly.

8. The screening method according to claim 7, wherein in said nano-emulsion:
- said cationic surfactant is selected from:
- *N*[1-(2,3-dioleyloxy) propyl]-*N*,*N*,*N-*trimethylammonium,
- 1,2-dioleyl-3-trimethylamonium-propane,
- *N-*(2-hydroxyethyl)-*N*,*N-*dimethyl-2,3-bis(tetradecyloxy-1-propananium),
- 1-[2-(oleoyloxy)ethyl]-2-oleyl-3-(2-hydroxyethyl)imidazolinium, and
- dioctadecylamidoglycylspermine; and/or
- said fusogenic lipid is 1,2-Dioleoyl-*sn*-Glycero-3-Phosphoethanolamine; and/or
- said amphiphilic lipid is a phospholipid.

9. The screening method according to claim 7 or 8, wherein, in said nano-emulsion, said co-surfactant is grafted with said biological ligand for targeting a cell and/or an organ.

10. The screening method according to any of claims 1 to 9, wherein said candidate molecule is a nucleotide sequence which may modulate RNA interfering mechanisms.

11. The screening method according to claim 10, wherein said nucleotide sequence which may modulate RNA interfering mechanisms is:
(i) a small interfering RNA (siRNA),
(ii) a locked nucleic acid (LNA), or
(iii) a microRNA (miRNA),
(iv) a long double-stranded RNA (RNAds).

12. The screening method according to any of claims 1 to 11, wherein said single DNA tag consists in a sequence of DNA, preferably double-stranded DNA, with at least 50 nucleotides or base pairs (bp) consisting in, on a strand and in the 5'-3' sense:
- a first sequence of at least 20 nucleotides common to all the single DNA tags of the library,
- a single sequence of at least 10 nucleotides specific to said molecule,
- a second sequence of at least 20 nucleotides common to all the single DNA tags of the library.

13. A screening method of a biomarker of a disease and/or of a phenotype feature comprising the following steps:
a) putting cells in contact with a library of synthetic nanoparticles, each nanoparticle being such as defined in any of claims 1 to 12, under conditions allowing said cells to integrate at least one of said nanoparticles,
b) selecting the cells having integrated the tracer and having a phenotype of interest,
c) identifying the candidate molecule having been integrated into the cell selected in step b) by identifying the sequence of said single DNA tag,
d) identifying as a biomarker of a disease and/or of a phenotype feature, the target of said molecule identified in step c).

14. The method according to claim 13, wherein said disease is a cancer or an infection.

15. The use of synthetic nanoparticles as defined in any of claims 1 to 12 for screening molecules of interest and/or of a biomarker of a disease and/or of a phenotype feature.

16. A library of synthetic nanoparticles as defined in any of claims 1 to 12.

17. A synthetic nanoparticle as defined in any of claims 1 to 12.

18. The synthetic nanoparticle according to claim 17, wherein said single DNA tag consists in a DNA sequence, preferably a double-strand DNA, with at least 50 nucleotides or base pairs (bp).
